# FASCICULE DE BREVET EUROPEEN

(11) **EP 0 977 749 B1**
(45) Date de publication et mention de la délivrance du brevet: **13.03.2002**
(21) Numéro de dépôt: 98930852.3
(22) Date de dépôt: 12.06.1998
(51) Int. Cl.: C07D 311/58, C07D 335/06, C07D 405/06, C07C 65/28, C07D 213/79, C07D 409/06, A61K 31/35

(54) **COMPOSES BI-AROMATIQUES ET COMPOSITIONS PHARMACEUTIQUES ET COSMETIQUES LES CONTENANT**
BI-AROMATISCHE VERBINDUNGEN UND DIESE ENTHALTENDE PHARMAZEUTISCHE UND KOSMETISCHE ZUSAMMENSTELLUNGEN
BI-AROMATIC COMPOUNDS AND PHARMACEUTICAL AND COSMETIC COMPOSITIONS CONTAINING SAME

(30) Priorité: 13.06.1997 FR 9707358
(43) Date de publication de la demande: 09.02.2000
(73) Titulaire: Galderma Research & Development, 06560 Valbonne (FR)
(72) Inventeur: BERNARDON, Jean-Michel, F-06650 Le Rouret (FR); TROUILLE, Simon, F-06650 Le Rouret (FR)
(74) Mandataire: Stalla-Bourdillon, Bernard
(86) Numéro de dépôt international: FR9801238
(87) Numéro de publication internationale: WO9856783

(56) Documents cités:
- EP-A- 0 661 258
- WO-A-93/16068
- WO-A-96/11902
- HIROYUKI KAGECHIKA ET AL.: "RETINOBENZOIC ACIDS 2." JOURNAL OF MEDICINAL CHEMISTRY., vol. 32, 1989, pages 834-840, XP000569539 WASHINGTON US

## Description

L'invention concerne, à titre de produits industriels nouveaux et utiles, des composés bi-aromatiques dont les noyaux aromatiques sont reliés par un radical divalent propynylène ou allénylène. Elle concerne également l'utilisation de ces nouveaux composés dans des compositions pharmaceutiques destinées à un usage en médecine humaine ou vétérinaire, ou bien encore dans des compositions cosmétiques.

Les composés selon l'invention ont une activité marquée dans les domaines de la différenciation et de la prolifération cellulaire et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

Il a déjà été décrit dans l'EP-661 258 des composés bi-aromatiques dont les noyaux aromatiques sont reliés par une liaison divalente propynylène, en tant que substances actives dans des compositions pharmaceutiques ou cosmétiques.

Les composés selon l'EP-661 258 répondent à la formule générale suivante : 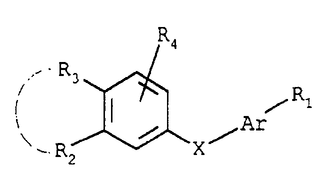 dans laquelle ;
Ar est un radical divalent aromatique éventuellement substitué par un radical R₅ ou hétéroaromatique éventuellement substitué par un radical R₆ lorsque l'hétéroatome est l'azote,
R₁ représente H, -CH₃, -CH₂OR₆, -OR₆, -COR₇ ou -S(O)ₜR₉, t étant 0, 1 ou 2,
R₂ et R₃ représentent H, alkyle en C₁-C₂₀, -OR₆ ou -SR₆,
ou R₂ et R₃ pris ensemble forment un cycle à 5 ou 6 chaînons éventuellement substitué par des groupes méthyles et/ou éventuellement interrompu par un atome d'oxygène ou de soufre,
R₄ et R₅ représentent H, un halogène, alkyle inférieur ou -OR₆,
R₆ représente H, alkyle inférieur ou -COR₉,
R₇ représente H, alkyle inférieur, 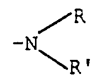 ou -OR₈,
R₈ représente H, alkyle, linéaire ou ramifié, en C₁-C₂₀, alkényle, mono- ou polyhydroxyalkyle, aryle ou aralkyle éventuellement substitué ou un reste de sucre ou d'aminoacide ou de peptide,
R₉ représente alkyle inférieur,
R et R' représentent H, alkyle inférieur, mono ou polyhydroxyalkyle, aryle éventuellement substitué ou un reste de sucre, d'aminoacide ou de peptide ou R et R' pris ensemble forment un hétérocycle, et
X représente un radical divalent qui, de droite à gauche ou inversement, a pour formule : 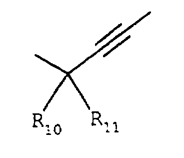 dans laquelle :
R₁₀ représente H, alkyle inférieur ou -OR₆,
R₁₁ représentant -OR₆,
ou R₁₀ et R₁₁ pris ensemble forment un radical oxo (=O), et les sels desdits composés de formule ci-dessus lorsque R₁ représente une fonction acide carboxylique et les isomères optiques et géométriques de ces dits composés.

Les composés selon la présente invention, par rapport à ceux de l'EP-661 258, se distinguent essentiellement par le fait que le substituant -X-Ar-R₁ est en position ortho du radical R₂ ou du cycle à 5 ou 6 chaînons lorsque R₂ et R₃ sont pris ensemble, alors que dans l'EP-661 258, le substituant -X-Ar-R₁ se trouve en position méta.

On a en effet constaté de façon inattendue et surprenante que cette modification de structure permettait d'en augmenter de façon significative les propriétés pharmaceutiques et cosmétiques et en outre d'en diminuer certains effets secondaires.

La présente invention a donc pour objet de nouveaux composés qui peuvent être représentés par la formule générale suivante : 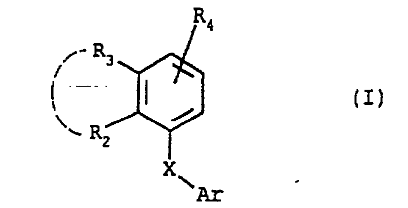 dans laquelle :
Ar représente un radical choisi parmi les formules (a) à (c) suivantes : 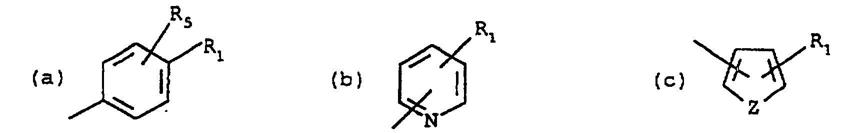 Z étant un atome d'oxygène ou de soufre,
R₁ représente -CH₃, -CH₂-O-R₆, -OR₆ ou -COR₇,
R₂ représente -OR₈, -SR₈ ou un radical ayant de 1 à 6 atomes de carbone et de 1 à 3 atomes d'oxygène ou de soufre si dans ce dernier cas R₄ représente alkyle, linéaire ou ramifié, en C₁-C₂₀ et est en position ortho ou méta par rapport à la liaison X-Ar,
R₃ représente alkyle en C₁-C₆, ou
R₂ et R₃ pris ensemble forment un cycle à 5 ou 6 chaînons éventuellement substitué par au moins un méthyle et/ou éventuellement interrompu par un atome d'oxygène ou de soufre,
R₄ représente H, un halogène, alkyle, linéaire ou ramifié, en C₁-C₂₀, -OR₈, un radical ayant de 1 à 6 atomes de carbone et de 1 à 3 atomes d'oxygène ou de soufre ou un radical aryle,
R₅ représente H, un halogène, alkyle, linéaire ou ramifié, en C₁-C₂₀ ou un radical -OR₈,
R₆ représente H, alkyle en C₁-C₆ ou un radical -COR₉,
R₇ représente H, alkyle en C₁-C₆, 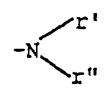 ou -OR₁₀,
R₈ représente H, alkyle en C₁-C₆ ou -COR₉,
R₉ représente alkyle en C₁-C₆
R₁₀ représente H, alkyle, linéaire ou ramifié, en C₁-C₂₀, alkényle, mono- ou polyhydroxyalkyle, aryle ou aralkyle éventuellement substitué(s) ou un reste de sucre,
r' et r" représentent H, alkyle en C₁-C₆, mono- ou polyhydroxyalkyle, aryle éventuellement substitué, un reste d'aminoacide ou de sucre ou pris ensemble avec l'atome d'azote forment un hétérocycle,
X représente un radical divalent qui, de droite à gauche ou inversement, a pour formule : 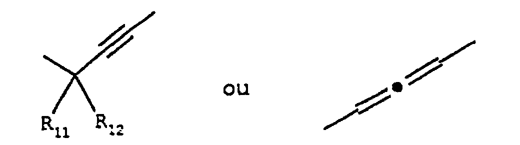
R₁₁ représentant H ou -OR₆, R₆ ayant la même signification que ci-dessus,
R₁₂ représentant H ou alkyle en C₁-C₆, ou
R₁₁ et R₁₂ pris ensemble forment un radical oxo (=O),
et les sels des composés de formule (I) lorsque R₁ représente une fonction acide carboxylique ainsi que les isomères optiques et géométriques desdits composés de formule (I).

Lorsque les composés selon l'invention se présentent sous forme d'un sel, il s'agit de préférence d'un sel d'un métal alcalin ou alcalino-terreux, ou encore de zinc ou d'une amine organique.

Selon la présente invention, on entend par alkyle en C₁-C₆, de préférence les radicaux méthyle, éthyle, isopropyle, butyle, tertiobutyle, et hexyle.

Par alkyle, linéaire ou ramifié, en C₁-C₂₀, on entend notamment les radicaux méthyle, éthyle, propyle, isopropyle, hexyle, heptyle,2-éthyl-hexyle, octyle, nonyle, dodécyle, hexadécyle et octadécyle.

Par monohydroxyalkyle, on entend un radical ayant de préférence 2 ou 3 atomes de carbone, notamment un radical 2-hydroxyéthyle 2-hydroxypropyle ou 3-hydroxypropyle.

Par polyhydroxyalkyle, on entend un radical ayant de préférence 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical ayant de 1 à 6 atomes de carbone et de 1 à 3 atomes d'oxygène ou de soufre, on entend les radicaux méthoxyméthyl éther, méthoxyéthoxyméthyl éther ou méthylthiométhyl éther.

Par aryle, on entend un radical pyridinique, un radical thiophényle ou un radical phényle éventuellement substitué par au moins un atome d'halogène, un hydroxyle, une fonction nitro, un alkyle inférieur, un radical CF3, un radical amino éventuellement protégé par une fonction acétyle ou éventuellement substitué par un ou deux alkyle(s) inférieur(s), un radical alkoxy ou un radical polyéther. On entend de préférence, par aryle un radical phényle éventuellement substitué par au moins un atome d'halogène, un hydroxyle, une fonction nitro, un alkyle inférieur, un radical CF3, un radical amino éventuellement protégé par une fonction acétyle ou éventuellement substitué par un ou deux alkyle(s) inférieur(s), un radical alkoxy ou un radical polyéther, ce dernier étant tel que défini ci-dessus.

Lorsque le substituant est un radical alkoxy, celui-ci est de préférence en C₁ - C₁₂ tels que notamment les radicaux méthoxy, éthoxy, propyloxy, isopropyloxy, hexyloxy, heptyloxy, octyloxy et nonyloxy.

Par aralkyle, on entend de préférence le radical benzyle ou phénéthyle éventuellement substitué par au moins un atome d'halogène, un hydroxyle ou une fonction nitro.

Par alkényle, on entend un radical ayant de préférence 1 à 5 atomes de carbone et présentant une ou plusieurs insaturations éthyléniques, tel que plus particulièrement le radical allyle.

Par reste de sucre, on entend un reste dérivant notamment du glucose, du galactose ou du mannose, ou bien encore de l'acide glucuronique.

Par reste d'aminoacide, on entend notamment un reste dérivant de la lysine, de la glycine ou de l'acide aspartique.

Par hétérocycle, on entend de préférence un radical pipéridino, morpholino, pyrrolidino ou pipérazino, éventuellement substitué en position 4 par un alkyle inférieur en C₁-C₆ ou un mono- ou polyhydroxyalkyle tels que définis ci-dessus.

Lorsque R₄ et R₅ représente un halogène, celui-ci est de préférence un atome de fluor, de chlore et de brome.

Selon une forme de réalisation préférée, les composés selon l'invention répondent à la formule générale suivante : 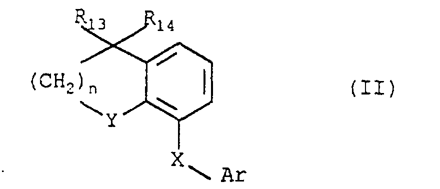 dans laquelle :
Ar représente un radical de formule (a) ou (b) suivante : 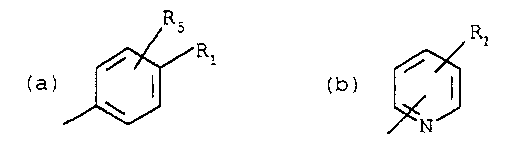
R₁ représente -COR₇,
R₅ et R₇ étant tels que définis ci-dessus pour la formule (I),
X représente un radical divalent qui, de droite à gauche ou inversement, a pour formule : 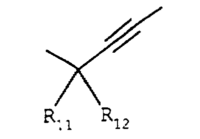
R₁₁ et R₁₂ représentent H,
R₁₃ et R₁₄, identiques ou différents, représentent H ou -CH₃,
Y représente un atome d'oxygène ou de soufre, un radical divalent méthylène, éthylidène ou isopropylidène, et
n est 1 ou 2.

Parmi les composés répondant aux formules (I) et (II) ci-dessus selon la présente invention, on peut notamment citer les suivants :
- 2-Hydroxy-4-[3-(4,4-diméthylchroman-8-yl)-prop-1-ynyl]benzoate de méthyle,
- Acide 2-hydroxy-4-[3-(4,4-diméthylchroman-8-yl)-prop-1-ynyl]benzoïque,
- 2-Hydroxy-4- [3-hydroxy-3-(4,4-diméthylchroman-8-yl)-prop-1-ynyl] benzoate de méthyle,
- Acide 2-hydroxy-4-[3-hydroxy-3-(4,4-diméthylchroman-8-yl)-prop-1-ynyl] benzoïque,
- 2-Hydroxy-4-[3-(4,4-diméthylthiochroman-8-yl)-prop-1-ynyl] benzoate de méthyle,
- Acide 2-hydroxy-4-[3-(4,4-diméthylthiochroman-8-yl)-prop-1-ynyl]benzoïque,
- 4-[3-Hydroxy-3-(5,5,8,8-tétraméthyl-3-phényl-5,6,7,8-tétrahydronaphthalèn-1-yl)-prop-1-ynyl]-benzoate d'éthyle,
- Acide 4-[3-hydroxy-3-(5,5,8,8-tétraméthyl-3-phényl-5,6,7,8-tétrahydro-naphthalèn-1-yl)-prop-1-ynyl]-benzoique,
- Acide 4-[3-(5,5,8,8-tétraméthyl-3-phényl-5,6,7,8-tétrahydro-naphthalèn-1-yl)-prop-1-ynyl]-benzoique,
- 4-[3-(4,4-diméthyl-thiochroman-5-yl)-3-hydroxy-prop-1-ynyl]-benzoate d'éthyle,
- Acide 4-[3-(4,4-diméthyl-thiochroman-5-yl)-3-hydroxy-prop-1-ynyl]-benzoïque,
- Acide 4-[3-(4,4-diméthyl-thiochroman-5-yl)-prop-1-ynyl]-benzoïque,
- 4- [3-(3,5-di-tert-butyl-2-méthoxyméthoxy-phényl)-3-hydroxy-prop-1-ynyl]-benzoate d'éthyle,
- Acide 4- [3-(3,5-di-tert-butyl-2-méthoxyméthoxy-phényl)-3-hydroxy-prop-1-ynyl]-benzoique,
- 4-[3-(3,5-di-tert-butyl-2-hydroxy-phényl)-3-hydroxy-prop-1-ynyl]-benzoate d'éthyle,
- 4-[3-(3,5-di-tert-butyl-2-hydroxy-phényl)-prop-1-ynyl]-benzoate d'éthyle,
- 4-[3-(3,5-di-tert-butyl-2-méthoxy-phényl)-3-hydroxy-prop-1-ynyl]-benzoate d'éthyle,
- Acide 4-[3-(3,5-di-tert-butyl-2-méthoxy-phényl)-3-hydroxy-prop-1-ynyl]-benzoïque,
- Acide 4-[3-(3,5-di-tert-butyl-2-méthoxy-phényl)-prop-1-ynyl]-benzoïque,
- 4-[3-(5-tert-butyl-4-méthoxy-biphényl-3-yl)-3-hydroxy-prop-1-ynyl]-benzoate d'éthyle,
- Acide 4-[3-(5-tert-butyl-4-méthoxy-biphényl-3-yl)-3-hydroxy-prop-1-ynyl]-benzoïque,
- 4-[3-(3,5-di-tert-butyl-2-méthoxy-phényl)-3-méthoxy-prop-1-ynyl]-benzoate d'éthyle,
- Acide 4-[3-(3,5-di-tert-butyl-2-méthoxy-phényl)-3-méthoxy-prop-1-ynyl]-benzoïque,
- 4-[3-(4,4-diméthyl-thiochroman-8-yl)-prop-1-ynyl]-benzoate de méthyle,
- 6-[3-(4,4-diméthyl-thiochroman-B-yl) -prop-1-ynyl]-nicotinate d'éthyle,
- 4-[3-(4,4-diméthyl-thiochroman-8-yl)-prop-1-ynyl)-benzaldéhyde,
- 4-[3-(4,4-diméthyl-thiochroman-8-yl)-prop-1-ynyl]-phénol,
- 4-[3-(5-tert-butyl-4-hydroxy-biphényl-3-yl)-3-hydroxy-prop-1-ynyl]-benzoate d'éthyle,
- Acide 4-[3-(5-tert-butyl-4-méthoxy-biphényl-3-yl)-prop-1-ynyl]-benzoïque,
- Acide 4-[3-(4,4-diméthylthiochroman-8-yl)-prop-1-ynyl]benzoïque,
- Acide 4-[3-(5,6,7,8-tétrahydro-5,5,8,8-tétraméthyl-1-naphtyl)-prop-1-ynyl] benzoïque,
- Acide 2-hydroxy-4-[3-(5,6,7,8-tétrahydro-5,5,8,8-tétraméthyl-1-naphtyl)-prop-1-ynyl]benzoïque,
- 2-Hydroxy-4-[3-hydroxy-3-(4,4-diméthylchroman-8-yl)-prop-1-ynyl] benzoate de méthyle,
- Acide 2-hydroxy-4-[3-hydroxy-3-(4,4-diméthylchroman-8-yl)-prop-1-ynyl] benzoïque,
- Acide 2-hydroxy-4-[3-(4,4-diméthylthiochroman-8-yl)-prop-1-ynyl]benzoïque,
- 4-[3-(4,4-diméthyl-thiochroman-8-yl)-prop-1-ynyl]-benzamide,
- N-éthyl-4-[3-(4,4-diméthyl-thiochroman-8-yl)-prop-1-ynyl]-benzamide,
- N-(4-hydroxyphényl)-4-[3-(4,4-diméthyl-thiochroman-8-yl)-prop-1-ynyl]-benzamide,
- Morpholide de l'acide 4-[3-(4,4-diméthyl-thiochroman-8-yl)-prop-1-ynyl]-benzoïque,
- Acide 4- [3-(4,4-diméthyl-thiochroman-8-yl)-prop-2-ynyl]-benzoïque,
- Acide 4-[3-(5,5,8,8-tetraméthyl-5,6,7,8-tetrahydro-naphthalen-1-yl)-prop-2-ynyl]-benzoïque,
- Acide 4-[3-(4,4-diméthyl-6-phényl-thiochroman-8-yl)-prop-1-ynyl]-benzoique,
- Acide 4-[3-(4,4-diméthyl-6-phényl-chroman-8-yl)-prop-1-ynyl]-benzoique,
- Acide 4-[3-(4,4-diméthyl-6-phényl-thiochroman-8-yl)-prop-2-ynyl]-benzoique,
- Acide 4-[3-(4,4-diméthyl-6-p-tolyl-thiochroman-8-yl)-prop-1-ynyl]-benzoique,
- Acide 4-[3-(5,5,8,8-tetraméthyl-5,6,7,8-tetrahydro-naphthalèn-1-yl)-prop-2-ynyl]-benzoique,
- Acide 4-[3-(5,5,8,8-tétraméthyl-3-p-tolyl-5,6,7,8-tétrahydro-naphthalèn-1-yl)-prop-1-ynyl]-benzoique,
- Acide 4-(3-[3-(4-méthoxy-phényl)-5,5,8,8-tétraméthyl-5,6,7,8-tétrahydro-naphthalèn-1-yl]-prop-1-ynyl)-benzoique,
- Acide 2-hydroxy-4-[3-(5,5,8,8-tétraméthyl-3-p-tolyl-5,6,7,8-tétrahydro-naphthalèn-1-yl) -prop-1-ynyl]-benzoique et
- Acide 3-hydroxy-4-[3-(5,5,8,8-tétramèthyl-3-phényl-5,6,7,8-tétrahydro-naphthalèn-1-yl)-prop-1-ynyl]-benzoique.

La présente invention a également pour objet les procédés de préparation des composés de formule (I) ci-dessus selon le schéma réactionnel donné au Tableau A.

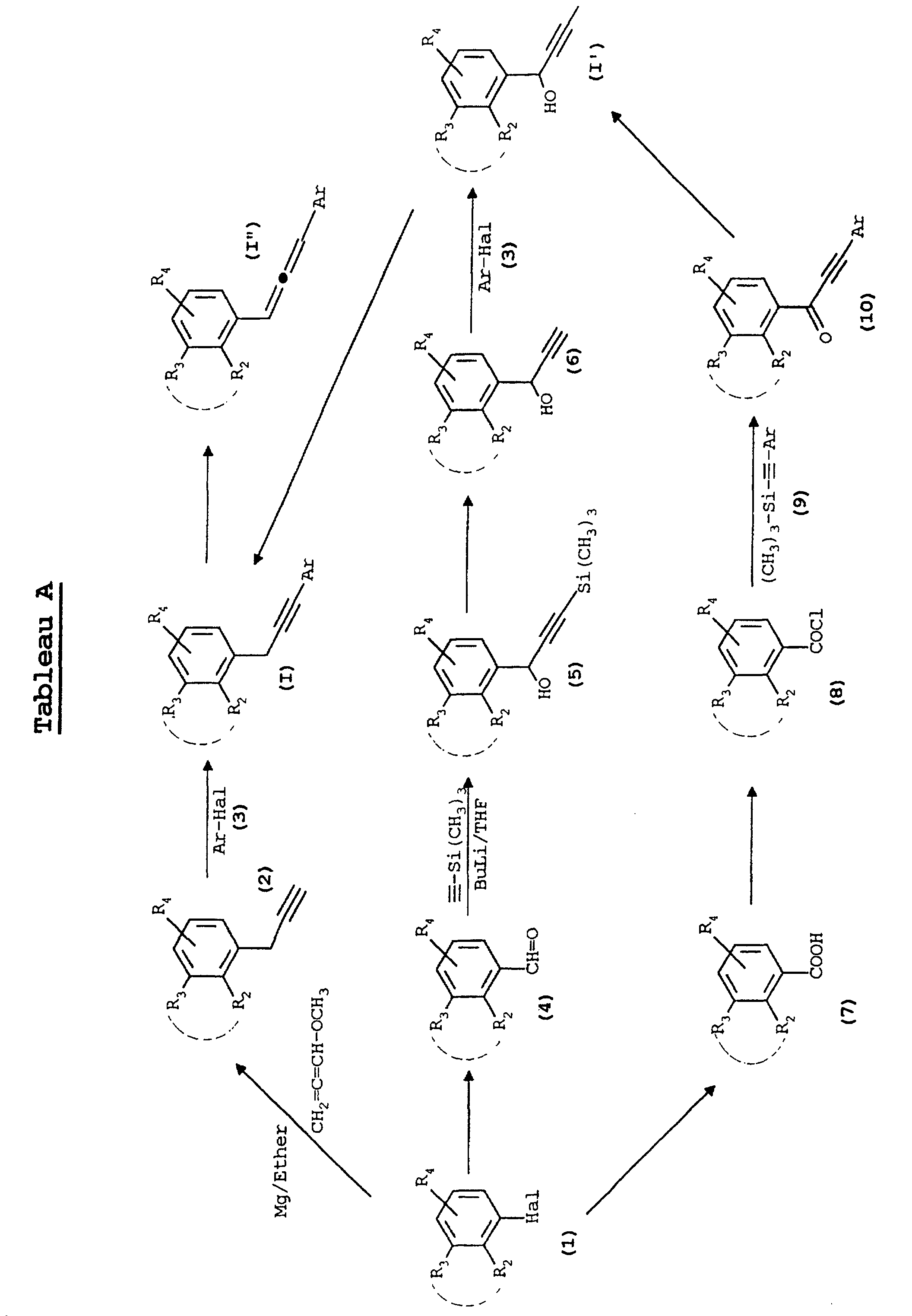

Les composés de formule (I) peuvent être préparés à partir d'un dérivé halogéné (1), de préférence bromé ou iodé, par transformation en dérivé magnésien puis réaction avec du méthoxyallène en présence de CuBr et obtention du dérivé propargylique (2). Ce dernier est ensuite couplé avec un dérivé halogéné (3), de préférence iodé ou bromé, en présence d'un catalyseur au palladium, par exemple le chlorure de bis-(triphénylphosphine)-palladium(II), dans un solvant, tel la triéthylamine.

Les composés de formule (I) peuvent être aussi préparés par une suite de réactions comprenant l'action du triméthylsilyl acétylénure de lithium sur un composé aldéhydique (4) et déprotection avec du fluorure de tétrabutylammonium dans le THF et obtention de l'alcool propargylique (6). Par couplage de ce dernier avec un dérivé halogéné (3), de préférence iodé ou bromé, en présence d'un catalyseur au palladium, par exemple le chlorure de bis-(triphénylphosphine)-palladium(II), et dans un solvant, tel la triéthylamine, on obtient le composé hydroxylé selon l'invention de formule (I'). Ce dernier, par réduction de la fonction alcool en carbure, en présence d'iodure de triméthylsilane dans un solvant tel l'hexane ou par transfert d'hydrure à partir d'un silane, tel le triéthylsilane, en présence de BF₃,Et₂O dans un solvant chloré tel le chlorure de méthylène conduit au composé de formule (I).

Les composés de formule (I) peuvent encore être préparés par une suite de réactions comprenant l'action d'un chlorure de benzoyle de formule (8) avec un dérivé acétylénique de formule (9) en présence d'un acide de Lewis (par exemple AlCl₃) dans un solvant chloré, tel le dichlorométhane. La cétone acétylénique (10) ainsi obtenue est réduite en composé hydroxylé selon l'invention (I') par action d'un hydrure alcalin, tel le borohydrure de sodium, dans un solvant alcoolique (par exemple le méthanol). La réduction de la fonction alcool de (I') en carbure est effectuée comme précédemment et conduit au composé de formule (I).

Les composés alléniques de formule (I") peuvent être préparés par chauffage des composés de formule (I) en présence d'une base (NaOH, Et₃N, DBU) dans un solvant tel l'heptane ou le THF.

Les produits de départ pour la synthèse des composés préférés de formule (II) peuvent être obtenus selon différents schémas réactionnels en fonction de la signification du radical Y.

Lorsque Y représente un atome de soufre, R₁₃ et R₁₄ représentant -CH₃ et n=2, c'est-à-dire des dérivés 4,4-diméthyl thiochromanyles, ceux-ci peuvent être obtenus à partir du 2-bromothiophénol par couplage avec le 4-bromo-2-méthyl-2-butène en présence de carbonate de potassium ou d'hydrure de sodium dans le DMF puis cyclisation soit en présence d'acide p-toluène sulfonique soit en présence de chlorure d'aluminium ou encore d'acide polyphosphorique selon le schéma réactionnel suivant :

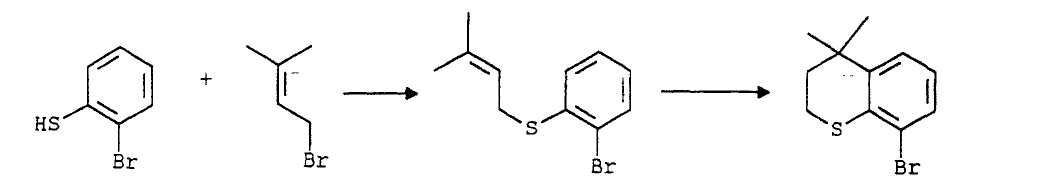

Lorsque Y représente un atome d'oxygène, R₁₃ et R₁₄ représentant -CH₃ et n=2, c'est-à-dire des dérivés 4,4-diméthyl chromanyles, ceux-ci peuvent être obtenus à partir du phénol par réaction avec le 3-méthyl-3-butèn-1-yl phosphate de diphényle en présence de chlorure stannique puis lithiation en présence de butyl-lithium et de tétraméthyléthylènediamine et réaction avec le diiodométhane (K. MCWILLIAMS, J. Org. Chem., **1966**, *61*, 7408-14) selon le schéma réactionnel suivant :

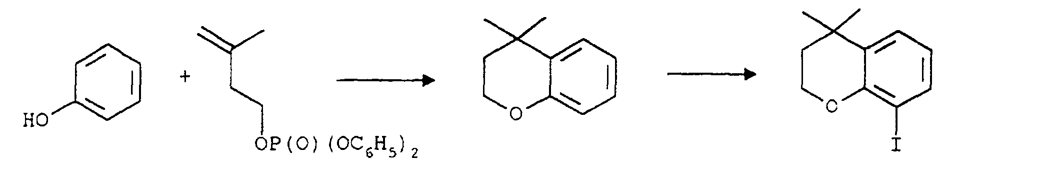

Lorsque Y représente un radical isopropylidène, R₁₃ et R₁₄ représentant -CH₃ et n=2, c'est-à-dire des dérivés tétrahydro-tétraméthylnaphtalényles, ceux-ci peuvent être obtenus à partir du 3-bromophénol par réaction avec le 2,5-dichloro-2,5-diméthylhexane en présence de chlorure d'aluminium puis hydrogénolyse en présence de palladium sur charbon et d'acide formique ou d'hydrogène et formation du dérivé triflate puis hydroformulation (H. KOTSUKI, Synthesis, **1996**, 470-2) selon le schéma réactionnel suivant :

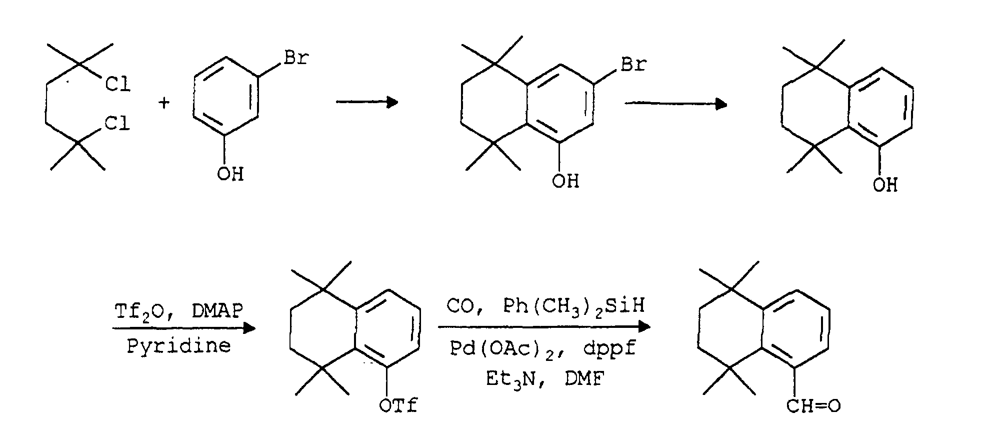

Lorsque Y représente un radical méthylène, R₁₃ et R₁₄ représentant -CH₃ et n=2 c'est-à-dire des dérivés tétrahydrodiméthyl naphtalényles, ceux-ci peuvent être obtenus à partir du 2-bromo-anisole par couplage avec le dérivé zincique du 1-bromo-4-méthylpent-3-ène en présence d'un catalyseur au palladium par exemple PdCl2/(dppf) (R.L. DANHEISER, J. Org. Chem., **1995**, *60*, 8341-8350) puis cyclisation en présence d'un acide de Lewis par exemple le chlorure d'aluminium, puis déméthylation avec BBr₃, formation du triflate et hydroformylation comme décrit ci-dessus.

Cette suite de réactions pouvant être représentée par le schéma réactionnel suivant :

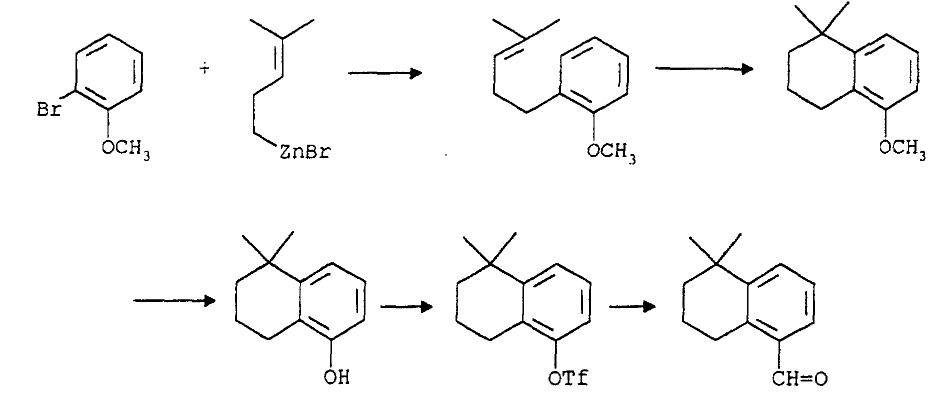

Lorsque R₁ représente -COOH, les composés sont préparés en protégeant R₁ par un groupe protecteur de type alkyle, allylique ou tert-butylique.

Le passage à la forme libre peut être effectué :
- dans le cas d'un groupe protecteur alkyle au moyen de soude ou d'hydroxyde de lithium dans un solvant alcoolique tel le méthanol ou dans le THF ;
- dans le cas d'un groupe protecteur allylique au moyen d'un catalyseur tel certains complexes de métaux de transition en présence d'une amine secondaire telle la morpholine ;
- dans le cas d'un groupe protecteur de type tert-butylique au moyen d'iodure de triméthylsilane.

Lorsque R₁ est -OH, les composés peuvent être obtenus à partir de l'acide correspondant par réduction en présence d'hydrure double de lithium et d'aluminium.

Lorsque R₁ est 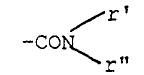 les composés peuvent être obtenus par transformation de l'acide correspondant en chlorure d'acide par exemple avec du chlorure de thionyle puis réaction avec l'ammoniaque ou une amine appropriée.

La présente invention a également pour objet à titre de médicament les composés de formule (I) telle que définie ci-dessus.

Ces composés présentent une activité dans le test de différenciation des cellules (F9) de tératocarcinome embryonnaire de souris (Cancer Research 43, p.5268, 1983) et/ou dans le test d'inhibition de l'ornithine décarboxylase après induction par le TPA chez la souris (Cancer Research 38, p.793-801, 1978). Ces tests montrent les activités des composés respectivement dans les domaines de la différenciation et de la prolifération cellulaire.

Dans le test de différenciation des cellules (F9), il est possible d'évaluer une activité agoniste, comme une activité antagoniste aux récepteurs de l'acide rétinoïque. En effet, un antagoniste est inactif lorsqu'il est seul dans ce test, mais inhibe partiellement ou totalement l'effet produit par un rétinoïde agoniste sur la morphologie et sur la sécrétion de l'activateur plasminogène. Ces composés présentent donc également une activité dans un test qui consiste à identifier des molécules antagonistes des RARs, tel que décrit dans la demande de brevet français n° 95-07302 déposée le 19 Juin 1995 par la Demanderesse. Ce test comprend les étapes suivantes : (i) on applique topiquement sur une partie de la peau d'un mammifère une quantité suffisante d'une molécule agoniste des RARs, (ii) on administre par voie systémique ou topique sur ce même mammifère ou sur cette même partie de la peau du mammifère, avant, pendant ou après l'étape (i), une molécule susceptible de présenter une activité antagoniste des RARs et (iii) on évalue la réponse sur la partie de la peau ainsi traitée du mammifère. Ainsi, la réponse à une application topique sur l'oreille d'un mammifère d'une molécule agoniste des RARs qui correspond à une augmentation de l'épaisseur de cette oreille peut être inhibée par l'administration par voie systémique ou topique d'une molécule antagoniste des RARs.

Les composés selon l'invention conviennent particulièrement bien dans les domaines des traitements suivants :
1) pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée telle que l'eczéma, ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photoinduit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acné ou la séborrhée simple,
11) pour traiter ou prévenir des états cancéreux ou précancéreux,
12) pour traiter les affections inflammatoires telles que l'arthrite,
13) pour traiter toute affection d'origine virale au niveau cutané ou général,
14) pour prévenir ou traiter l'alopécie,
15) pour traiter les affections dermatologiques ou générales à composante immunologique, et
16) pour traiter les affections du système cardiovasculaire telles que l'artériosclérose.

Dans les domaines thérapeutiques mentionnés ci-dessus, les composés selon l'invention peuvent être avantageusement employés en combinaison avec d'autres composés à activité de type rétinoide, avec les vitamines D ou leurs dérivés, avec des corticostéroides, avec des anti-radicaux libres, des α-hydroxy ou α-cétoacides ou leurs dérivés ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutase ou SOD, l'Ubiquinol ou certains chélatants de métaux. Par α-hydroxy ou α-céto acides ou leurs dérivés, on entend par exemple l'acide lactique, l'acide malique, l'acide citrique, l'acide glycolique, l'acide mandélique, l'acide tartrique, l'acide glycérique ou l'acide ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions pharmaceutiques contenant au moins un composé de formule (I) telle que définie ci-dessus, l'un de ses isomères optiques ou géométriques ou un de ses sels.

Les compositions pharmaceutiques sont destinées notamment au traitement des affections susmentionnées, et sont caractérisées par le fait qu'elles contiennent un support pharmaceutiquement acceptable et compatible avec le mode d'administration retenu, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les compositions peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/kg en poids corporel, et ceci à raison de 1 à 3 prises.

Par voie topique, les compositions pharmaceutiques à base des composés selon l'invention sont plus particulièrement destinées au traitement de la peau et des muqueuses et peuvent alors se présenter sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée du principe actif. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photoinduit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroides, avec des anti-radicaux libres, des α-hydroxy ou α-céto acides ou leurs dérivés, ou bien encore avec des bloqueurs de canaux ioniques tous ces derniers composés étant tels que définis ci-dessus.

La présente invention a donc également pour objet une composition cosmétique qui est caractérisée par le fait qu'elle contient dans un support cosmétiquement acceptable, au moins un composé de formule (I) telle que définie ci-dessus ou l'un de ses isomères optiques ou géométriques ou l'un de ses sels, ladite composition cosmétique pouvant notamment se présenter sous forme d'une crème, d'un lait, d'une lotion, d'un gel, de microsphères ou de nanosphères ou de vésicules lipidiques ou polymériques, d'un savon ou d'un shampooing.

La concentration en composé de formule (I) dans les compositions cosmétiques selon l'invention est avantageusement comprise entre 0,001% et 3% en poids par rapport au poids total de la composition.

Les compositions pharmaceutiques et cosmétiques selon l'invention peuvent en outre contenir des additifs inertes ou même pharmacodynamiquement ou cosmétiquement actifs ou des combinaisons de ces additifs et notamment : des agents mouillants, des agents dépigmentants tels que l'hydroquinone, l'acide azélaïque, l'acide caféique ou l'acide kojique ; des émollients ; des agents hydratants comme le glycérol, le PEG 400, la thiamorpholinone et ses dérivés ou bien encore l'urée ; des agents antiséborrhéiques ou antiacnéiques, tels que la S-carboxyméthylcystéine, la S-benzyl-cystéamine, leurs sels ou leurs dérivés, ou le peroxyde de benzoyle ; des antibiotiques comme l'érythromycine et ses esters, la néomycine, la clindamysine et ses esters, les tétracyclines ; des agents antifongiques tels que le kétokonazole ou les polyméthylène-4,5 isothiazolidones-3 ; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoin (5,4-diphénylimidazolidine 2,4-dione) ; des agents anti-inflammatoires non stéroidiens ; des caroténoïdes et, notamment le β-carotène ; des agents anti-psoriatiques tels que l'anthraline et ses dérivés ; et enfin les acides eicosa-5,8,11,14-tétraynoique et eicosa-5,8,11-trynoique, leurs esters et amides.

Les compositions selon l'invention peuvent également contenir des agents d'amélioration de la saveur, des agents conservateurs tels que les esters de l'acide parahydroxybenzoique, des agents stabilisants, des agents régulateurs d'humidité, des agents régulateurs de pH, des agents modificateurs de pression osmotique, des agents émulsionnants, des filtres UV-A et UV-B, des antioxydants tels que l'α-tocophérol, le butylhydroxyanisole ou le butylhydroxytoluène.

On va maintenant donner à titre d'illustration et sans aucun caractère limitatif, plusieurs exemples de préparation des composés actifs de formule (I) selon l'invention, ainsi que diverses formulations pharmaceutiques et cosmétiques à base de ces composés.

### EXEMPLE 1

### 2-Hydroxy-4-[3-(4,4-diméthylchroman-8-yl)-prop-1-ynyl]benzoate de méthyle.

### (a) 4,4-diméthyl-8-iodochromane.

Dans un tricol et sous argon, on introduit 2,00 g (12,3 mmoles) de 4,4-diméthylchromane et 30 ml d'éther éthylique. On ajoute goutte à goutte 2,4 ml (15,9 mmoles) de tétraméthyléthylènediamine (TMEDA), refroidit à -78°C et ajoute goutte à goutte 5,9 ml (14,8 mmoles) de n-butyllithium (2,5 M dans l'hexane). On laisse remonter la température à -20°C en deux heures, puis à température ambiante et agite pendant douze heures. Dans un autre tricol et sous argon, on introduit 1,3 ml (16,0 mmoles) de diiodométhane et 15 ml d'éther éthylique. On refroidit à 0°C et introduit la solution précédente préalablement refroidie à -78°C, puis laisse remonter à la température ambiante et agite pendant douze heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice, élué avec de l'heptane. Après évaporation des solvants, on recueille 1,30 g (37%) du composé attendu, sous la forme d'une huile jaune pâle.
1H NMR (CDCl3) d 1,32 (s, 6H), 1,84 (t, 2H, J = 5,4 Hz), 4,28 (t, 2H, J = 5,4 Hz), 6,62 (t, 1H, J = 7,7 Hz), 7,24 (dd, 1H, J = 7,8 / 1,5 Hz), 7,56 (dd, 1H, J = 7,7 / 1,5 Hz).

### (b) méthoxyallène.

Dans un tricol et sous argon, on introduit 210 ml (2,5 moles) de propargyl méthyléther et 12,00 g (110,0 mmoles) de tert-butylate de potassium. On chauffe à reflux pendant trois heures et distille le milieu réactionnel sous la pression atmosphérique. On recueille la fraction passant à 51°C pour obtenir 153,50 g (88%) du composé attendu, sous la forme d'une huile incolore.
1H NMR (CDCl3) d 3,41 (s, 3H), 5,48 (d, 2H, J = 5,9 Hz), 6,77 (t, 1H, J = 5,9 Hz).

### (c) 3-(4,4-diméthylchroman-8-yl)-prop-1-yne.

Dans un tricol et sous argon, on introduit 280 mg (11,5 mmoles) de magnésium activé par 1 goutte de dibromoéthane. On ajoute goutte à goutte une solution de 3,00 g (10,4 mmoles) de 4,4-diméthyl-8-iodochromane dans 15 ml d'éther éthylique de manière à maintenir le reflux du solvant, et agite à 35°C pendant quinze minutes. On refroidit ensuite le milieu réactionnel à -5°C, ajoute 40 mg (0,2 mmole) de CuI et introduit goutte à goutte une solution composée de 1,24 g (17,7 mmoles) de methoxyallène dans 5 ml d'éther éthylique. On agite pendant une heure à -5°C, laisse remonter à la température ambiante et agite deux heures. On verse le milieu réactionnel sur une solution saturée de chlorure d'ammonium, extrait avec de l'acétate d'éthyle, décante la phase organique, sèche sur sulfate de magnésium, évapore. Le résidu obtenu est purifié par chromatographie sur colonne de silice, élué avec de l'heptane. Après évaporation des solvants, on recueille 1,30 g (65%) du composé attendu, sous la forme d'une huile jaune.
1H NMR (CDCl3) d 1,33 (s, 6H), 1,83 (t, 2H, J = 5,4 Hz), 2,15 (t, 1H, J = 2,7 Hz), 3,52 (d, 2H, J = 2,7 Hz), 4,21 (t, 2H, J = 5,4 Hz), 6,87 (t, 1H, J = 7,6 Hz), 7,18 (dd, 1H, J = 7,9 / 1,5 Hz), 7,33 (dd, 1H, J = 7,4 / 1,5 Hz).

### (d) 2-hydroxy-4-[3-(4,4-diméthylchroman-8-yl)-prop-1-ynyl]benzoate de méthyle.

Dans un tricol et sous argon, on introduit 1,18 g (5,9 mmoles) de 3-(4,4-diméthylchroman-8-yl)-1-propyne, 1,60 g (5,9 mmoles) de 2-hydroxy-4-iodobenzoate de méthyle et 60 ml de triéthylamine. On dégaze le milieu réactionnel par barbotage d'azote, et introduit 332 mg (0,46 mmole) de chlorure de bis(triphénylphosphine) palladium(II), 134 mg d'iodure de cuivre et agite à la température ambiante pendant huit heures. On évapore à sec le milieu réactionnel, reprend par de l'acétate d'éthyle et de l'acide chlorhydrique (1N), décante la phase organique, sèche sur sulfate de magnésium, évapore. Le résidu est purifié par chromatographie sur colonne de silice, élué avec l'heptane. Après évaporation des solvants, on obtient une huile qui cristallise lentement et que l'on recristallise dans l'heptane. On recueille 1,00 g (50%) de 2-hydroxy-4-[3-(4,4-diméthylchroman-8-yl)-prop-1-ynyl]benzoate de méthyle, sous la forme d'un solide blanc de point de fusion 92-93°C.
1H NMR (CDCl3) d 1,34 (s, 6H), 1,84 (t, 2H, J = 5,4 Hz), 3,75 (s, 2H), 3,94 (s, 3H), 4,23 (t, 2H, J = 5,4 Hz), 6,89 (t, 1H, J = 7,6 Hz), 6,95 (dd, 1H, J = 8,2 / 1,5 Hz), 7,06 (d, 1H, J = 1,4 Hz), 7,20 (d, 1H, J = 6,3 Hz), 7,35 (d, 1H, J = 7,4 Hz), 7,75 (d, 1H, J = 8,2 Hz), 10,73 (s, 1H).

### EXEMPLE 2

### Acide 2-hydroxy-4-[3-(4,4-diméthylchroman-8-yl)-prop-1-ynyl] benzoïque.

Dans un ballon, on introduit 860 mg (2,5 mmoles) de l'ester méthylique obtenu à l'exemple 1(d), 1,00 g (25,0 mmoles) d'hydroxyde de lithium et 50 ml de THF. On chauffe à reflux pendant 18 heures et évapore à sec le milieu réactionnel. On reprend le résidu par de l'eau, acidifie à pH 1, extrait avec de l'éther éthylique, décante la phase organique, sèche sur sulfate de magnésium, évapore. On triture le résidu dans l'heptane, filtre et recueille 560 mg (70%) d'acide 2-hydroxy-4-[3-(4,4-diméthylchroman-8-yl)-prop-1-ynyl]benzoique, sous la forme d'un solide blanc de point de fusion 182-183°C.
1H NMR (DMSO D6) d 1,29 (s, 6H), 1,79 (t, 2H, J = 5,2 Hz), 3,72 (s, 2H), 4,20 (t, 2H, J = 5,3 Hz), 6,67 (t, 1H, J = 7,6 Hz), 6,76 à 6,79 (m, 2H), 7,05 (d, 2H, J = 7,6 Hz), 7,55 (d, 1H, J = 8,6 Hz).

### EXEMPLE 3

### 2-Hydroxy-4-[3-hydroxy-3-(4,4-diméthylchroman-8-yl)-prop-1-ynyl] benzoate de méthyle.

### (a) 4,4-diméthylchroman-8-yl-carboxaldéhyde.

Dans un tricol et sous courant d'azote, on introduit 14,40 g (50,0 mmoles) de 4,4-diméthyl-8-iodochromane et 50 ml de THF. A - 78°C, on ajoute goutte à goutte 22 ml (55,0 mmoles) de n-butyllithium (2,5 M dans l'hexane), agite 30 minutes puis ajoute 4,2 ml (55,0 mmoles) de DMF et laisse remonter à la température ambiante. On verse le milieu réactionnel sur une solution aqueuse de chlorure d'ammonium, extrait avec de l'éther éthylique, décante la phase organique, sèche sur sulfate de magnésium, évapore. On recueille 10,40 g (100%) du composé attendu, sous la forme d'une huile jaune.
1H NMR (CDCl3) d 1,36 (s, 6H), 1,86 (t, 2H, J = 5,2 Hz), 4,29 (t, 2H, J = 5,6 Hz), 6,91 (t, 1H, J = 8,4 Hz), 7,50 (dd, 1H, J = 7,7 / 1,7 Hz), 7,64 (dd, 1H, J = 7,9 / 1,7 Hz), 10,42 (s, 1H).

### (b) α-éthynyl-4,4-diméthylchroman-8-yl-méthanol.

Dans un tricol, on introduit 7,6 ml (54,0 mmoles) de triméthylsilylacétylène et 50 ml de THF. A -78°C et sous courant d'azote, on ajoute goutte à goutte une solution de 22 ml (54,0 mmoles) de n-butyllithium (2,5 M dans l'hexane) et laisse revenir à la température ambiante. Le milieu réactionnel est introduit goutte à goutte dans une solution froide (-78°C), composée de 9,30 g (49,0 mmoles) de 4,4-diméthylchroman-8-yl-carboxaldéhyde et de 50 ml de THF. On laisse le milieu réactionnel revenir à la température ambiante, verse sur une solution aqueuse de chlorure d'ammonium, extrait avec de l'éther éthylique, décante la phase organique, sèche sur sulfate de magnésium, évapore. On obtient 14,00 g (100%) du composé attendu, sous la forme d'une huile jaune. 3,00 g (10,0 mmoles) de cette huile sont mélangés avec 50 ml de THF, et on ajoute goutte à goutte 11,5 ml (12,6 mmoles) d'une solution de fluorure de tétrabutylammonium (1,1 M dans le THF). On agite à la température ambiante pendant une heure, verse le milieu réactionnel dans l'eau, extrait avec de l'éther éthylique, décante la phase organique, sèche sur sulfate de magnésium, évapore. On recueille 2,30 g (100%) du composé attendu, sous la forme d'une huile incolore.
1H NMR (CDCl3) d 1,33 (s, 6H), 1,81 à 1,88 (m, 2H), 2,59 (d, 1H, J = 2,2 Hz), 4,11 (d, 1H, J = 6,1 Hz), 4,25 (t, 2H, J = 4,7 Hz), 5,68 (dd, 1H, J = 6,1 / 2,0 Hz), 6,90 (t, 1H, J = 7,7 Hz), 7,25 (d, 1H, J = 7,8 Hz), 7,41 (d, 1H, J = 7,7 Hz).

### (c) 2-hydroxy-4-[3-hydroxy-3-(4,4-diméthylchroman-8-yl)-prop-1-ynyl] benzoate de méthyle.

Dans un tricol, on introduit 3,00 g (13,9 mmoles) d'α-éthynyl-4,4-diméthyl chroman-8-yl-méthanol, 3,90 g (13,9 mmoles) de 2-hydroxy-4-iodobenzoate de méthyle et 100 ml de triéthylamine. On dégaze le milieu réactionnel avec de l'azote pendant 30 minutes, puis ajoute successivement 780 mg (1,1 mmole) de chlorure de bis (triphénylphosphine)palladium(II) et 320 mg (1,7 mmole) d'iodure de cuivre. On agite à la température ambiante pendant quatre heures, évapore à sec le milieu réactionnel, reprend le résidu obtenu par de l'eau et de l'éther éthylique. On décante la phase organique, sèche sur sulfate de magnésium, évapore. Le résidu obtenu est purifié par chromatographie sur colonne de silice, élué avec un mélange composé de 50% d'acétate d'éthyle et de 50% d'heptane. On recueille 2,85 g (56%) de 2-hydroxy-4-[3-hydroxy-3-(4,4-diméthylchroman-8-yl)-prop-1-ynyl] benzoate de méthyle, sous la forme d'un solide blanc de point de fusion 122-123°C.
1H NMR (CDCl3) d 1,36 (s, 6H), 1,87 à 1,90 (m, 2H), 3,18 (d, 1H, J = 6,4 Hz), 3,95 (s, 3H), 4,31 (t, 2H, J = 5,3 Hz), 5,86 (d, 1H, J = 6,4 Hz), 6,89 à 7,00 (m, 2H), 7,09 (s, 1H), 7,29 (d, 1H, J = 7,9 Hz), 7,40 (d, 1H, J = 7,4 Hz), 7,77 (d, 1H, J = 8,2 Hz).

### EXEMPLE 4

### Acide 2-hydroxy-4-[3-hydroxy-3-(4,4-diméthylchroman-8-yl)-prop-1-ynyl] benzoïque.

Dans un ballon, on introduit 2,80 g (7,6 mmoles) du composé obtenu à l'exemple 3(c), 3,20 g (76,5 mmoles) d'hydroxyde de lithium et 100 ml de THF. On chauffe à reflux pendant 18 heures et évapore à sec le milieu réactionnel. On reprend le résidu par de l'eau, acidifie à pH 1, extrait avec de l'éther éthylique, décante la phase organique, sèche sur sulfate de magnésium, évapore. On triture le résidu dans l'heptane, filtre et recueille 660 mg (25%) d'acide 2-hydroxy-4-[3-hydroxy-3-(4,4-diméthylchroman-8-yl)-prop-1-ynyl] benzoïque, sous la forme d'un solide blanc de point de fusion 225-227°C.
1H NMR (CDCl3 + 2 gouttes de DMSO D6) d 1,34 (s, 6H), 1,87 (t, 2H, J = 6,0 Hz), 3,50 (s, 1H), 4,28 (t, 2H, J = 5,7 Hz), 5,90 (s, 1H), 6,88 à 6,96 (m, 2H), 7,02 (s, 1H), 7,27 (d, 1H, J = 7,8 Hz), 7,46 (d, 1H, J = 7,4 Hz), 7,79 (d, 1H, J = 8,1 Hz), 11,23 (br s, 1H).

### EXEMPLE 5

### 2-Hydroxy-4-[3-(4,4-diméthylthiochroman-8-yl)-prop-1-ynyl] benzoate de méthyle.

### (a) 2-bromo-1-(3-méthylbut-2-ènylthio)benzène.

Dans un tricol, on introduit 19,30 g (102,0 mmoles) de 2-bromothiophénol, 160 ml de DMF et 15,50 g (112,0 mmoles) de carbonate de potassium. On ajoute goutte à goutte 13 ml (112,0 mmoles) de 1-bromo-3-méthyl-2-butène et agite à la température ambiante pendant deux heures. On verse le milieu réactionnel dans l'eau, extrait avec de l'acétate d'éthyle, décante la phase organique, lave à l'eau, sèche sur sulfate de magnésium, évapore. On recueille 26,00 g (99%) du composé attendu, sous la forme d'une huile orangée.
1H NMR (CDCl3) d 1,65 (s, 3H), 1,73 (s, 3H), 3,56 (d, 2H, J = 7,7 Hz), 5,32 (td, 1H, J = 7,7 / 1,4 Hz), 6,96 à 7,06 (m, 1H), 7,22 à 7,26 (m, 2H), 7,52 (d, 1H, J = 7,7 Hz).

### (b) 4,4-diméthyl-8-bromothiochromane.

Dans un tricol, on introduit 26,00 g (102,0 mmoles) de 2-bromo-1-(3-méthylbut-2-ènylthio)benzène, 180 ml de toluène et 23,20 g (122,0 mmoles) d'acide para-toluène sulfonique. On chauffe à reflux pendant quatre heures et évapore le milieu réactionnel à sec. On reprend par une solution aqueuse d'hydrogénocarbonate de sodium, extrait avec de l'acétate d'éthyle, décante la phase organique, sèche sur sulfate de magnésium, évapore. Le résidu obtenu est purifié par chromatographie sur colonne de silice, élué avec de l'heptane. On recueille 20,00 g (76%) du composé attendu, sous la forme d'une huile orangée.
1H NMR (CDCl3) d 1,33 (s, 6H), 1,94 (t, 2H, J = 6,0 Hz), 3,04 (t, 2H, J = 6,1 Hz), 6,89 (t, 1H, J = 7,9 Hz), 7,34 (d, 2H, J = 7,9 Hz).

### (c) 3-(4,4-diméthylthiochroman-8-yl)-prop-1-yne.

De manière analogue à l'exemple 1(c), à partir de 3,00 g (11,7 mmoles) de 4,4-diméthyl-8-bromothiochromane, on obtient 710 mg (28%) du composé attendu, sous la forme d'une huile jaune pâle.
1H NMR (CDCl3) d 1,34 (s, 6H), 1,95 (t, 2H, J = 6,1 Hz), 2,23 (t, 1H, J = 2,7 Hz), 3,04 (t, 2H, J = 6,2 Hz), 3,53 (d, 2H, J = 2,6 Hz), 7,05 (t, 1H, J = 7,7 Hz), 7,32 (d, 1H, J = 7,8 Hz), 7,38 (d, 1H, J = 7,7 Hz).

### (d) 2-hydroxy-4-[3-(4,4-diméthylthiochroman-8-yl)-prop-1-ynyl]benzoate de méthyle.

Dans un tricol et sous argon, on introduit 670 mg (3,1 mmoles) de 3-(4,4-diméthylthiochroman-8-yl)-prop-1-yne, 860 mg (3,1 mmoles) de 2-hydroxy-4-iodo benzoate de méthyle et 33 ml de triéthylamine. On dégaze le milieu réactionnel par barbotage d'azote, et introduit 174 mg (0,25 mmole) de bis(triphénylphosphine) palladium(II)chlorure, 71 mg d'iodure de cuivre et agite à la température ambiante pendant huit heures. On évapore à sec le milieu réactionnel, reprend par de l'acétate d'éthyle et de l'acide chlorhydrique (1N), décante la phase organique, sèche sur sulfate de magnésium, évapore. Le résidu est purifié par chromatographie sur colonne de silice, élué avec un mélange composé de 99% d'heptane et de 1% d'acétate d'éthyle. Après évaporation des solvants, on recueille 1,50 g (75%) de 2-hydroxy-4-[3-(4,4-diméthylthiochroman-8-yl)-prop-1-ynyl] benzoate de méthyle, sous la forme d'une huile jaune.
1H NMR (CDCl3) d 1,35 (s, 6H), 1,97 (t, 2H, J = 6,0 Hz), 3,06 (t, 2H, J = 6,1 Hz), 3,76 (s, 2H), 3,94 (s, 3H), 6,96 (dd, 1H, J = 8,2 / 1,5 Hz), 7,04 à 7,10 (m, 2H), 7,33 (d, 1H, J = 6,9 Hz), 7,41 (d, 1H, J = 7,4 Hz), 7,75 (d, 1H, J = 8,2 Hz).

### EXEMPLE 6

### Acide 2-hydroxy-4-[3-(4,4-diméthylthiochroman-8-yl)-prop-1-nyl] benzoïque.

De manière analogue à l'exemple 2, à partir de 1,40 g (3,8 mmoles) du composé obtenu à l'exemple 5(d), on obtient 960 mg (70%) d'acide 2-hydroxy-4-[3-(4,4-diméthylthiochroman-8-yl)-prop-1-ynyl]benzoïque, sous la forme d'un solide blanc de point de fusion 190-191°C.
1H NMR (DMSO D6) d 1,29 (s, 6H), 1,69 (t, 2H, J = 5,9 Hz), 3,04 (t, 2H, J = 6,0 Hz), 3,75 (s, 2H), 6,96 à 6,99 (m, 2H), 7,06 (t, 1H, J = 7,7 Hz), 7,32 (d, 1H, J = 7,3 Hz), 7,38 (d, 1H, J = 7,8 Hz), 7,76 (d, 1H, J = 8,4 Hz).

### EXEMPLE 7

### 4-[3-hydroxy-3-(5,5,8,8-tétraméthyl-3-phényl-5,6,7,8-tétrahydro-naphthalèn-1-yl)-prop-1-ynyl]-benzoate d'éthyle.

### (a) 3-bromo-5,5,8,8-tétraméthyl-5,6,7,8-tétrahydro-naphthalèn-1-ol.

Dans un tricol, sous atmosphère d'argon, on introduit 13,40 g (100,0 mmoles) de chlorure d'aluminium, et 100 ml de dichlorométhane. On ajoute goutte à goutte une solution composée de 34,60 g (199,0 mmoles) de 3-bromophénol, 89,00 g (486,0 mmoles) de dichloro-2,5-diméthyl-2,5-hexane et 300 ml de dichlorométhane. Le milieu réactionnel est agité pendant seize heures à la température ambiante. On verse le milieu réactionnel dans l'eau, extrait par du dichlorométhane, lave à l'eau, décante la phase organique, sèche sur sulfate de magnésium, évapore. Le résidu obtenu est purifié par chromatographie sur colonne de silice, élué par un mélange composé de 80% d'heptane et de 20% de dichlorométhane. Après évaporation des solvants, on recueille 30,00 g (53%) du composé attendu, sous la forme de cristaux blancs de point de fusion 93°C.
1H NMR (CDCl3) d 1,25 (s, 6H), 1,38 (s, 6H), 1,57 à 1,69 (m, 4H), 4,78 (s, 1H), 6,64 (d, 1H, J = 2,0 Hz), 7,04 (d, 1H, J = 2,0 Hz).

### (b) 5,5,8,8-tétraméthyl-3-phényl-5,6,7,8-tétrahydro-naphthalèn-1-ol.

Dans un tricol, on introduit 12,93 g (106,0 mmoles) d'acide phényl boronique, 20,00 g (70,7 mmoles) du composé obtenu à l'exemple 7(a), 400 ml de DME et 70 ml d'une solution aqueuse de carbonate de potassium (2M). On dégaze le milieu réactionnel par barbotage d'argon et ajoute 4,08 g (3,5 mmoles) de tetrakistriphénylphosphinepalladium(0) et chauffe à 90°C pendant huit heures. On verse le milieu réactionnel dans l'eau, extrait par de l'acétate d'éthyle, décante la phase organique, sèche sur sulfate de magnésium, évapore. Le résidu obtenu est purifié par chromatographie sur colonne de silice, élué par de l'heptane. Après évaporation des solvants, on recueille 13,44 g (68%) du composé attendu, sous la forme d'une poudre blanche de point de fusion 121°C.
1H NMR (CDCl3) d 1,33 (s, 6H), 1,46 (s, 6H), 1,65 à 1,73 (m, 4H), 4,77 (s, 1H), 6,69 (d, 1H, J = 1,8 Hz), 7,16 (d, 1H, J = 1,8 Hz), 7,24 à 7,52 (m, 3H), 7,53 (d, 2H, J = 8,5 Hz).

### (c) trifluoro-méthanesulfonate de 5,5,8,8-tétraméthyl-3-phényl-5,6,7,8-tétrahydro-naphthalèn-1-yle.

Dans un tricol et sous argon, on introduit 13,44 g (47,9 mmoles) du composé obtenu à l'exemple 7(b), 100 ml de dichlorométhane et 9,95 g (81,5 mmoles) de N,N-diméthy-4-aminopyridine. On refroidit à 0°C et ajoute goutte à goutte 12,1 ml (71,9 mmoles) d'anhydride triflique. La température est remontée naturellement à la température ambiante en seize heures, et le milieu réactionnel est évaporé à sec. On ajoute de l'acétate d'éthyle, et acidifie à pH 3 avec de l'acide chlorhydrique 1N. Le produit est extrait avec de l'acétate d'éthyle, la phase organique lavée à l'eau puis à l'aide d'une solution saturée de chlorure de sodium, séchée sur sulfate de magnésium, filtrée et les solvants évaporés. On obtient 19,29 g (97%) du composé attendu, sous la forme d'une poudre blanche de point de fusion 110°C.
1H NMR (CDCl3) d 1,35 (s, 6H), 1,45 (s, 6H), 1,71 (s, 4H), 7,35 à 7,55 (m, 7H).

### (d) 5,5,8,8-tétraméthyl-3-phényl-5,6,7,8-tétrahydro-naphthalène-1-carboxylate de méthyle.

Dans une bombe à hydrogéner, on introduit 16,12 g (39,1 mmoles) du triflate obtenu à l'exemple 7(c), 1,61 g (3,9 mmoles) de 1,3-bis(diphénylphosphino)propane (DPPP), 440 mg (1,9 mmole) d'acétate de palladium, 130 ml de DMF, 10,9 ml (78,2 mmoles) de triéthylamine et 17,1 ml (390,8 mmoles) de méthanol. Le milieu réactionnel est confiné sous une pression de quatre bars de monoxyde de carbone et chauffé sous agitation à 70°C pendant sept heures. Le mélange est refroidi, évoporé au maximum, repris par une solution saturée de chlorure de sodium, extrait par de l'acétate d'éthyle, lavé à l'aide d'une solution diluée d'acide chlorhydrique, puis à l'eau, la phase organique est séchée sur sulfate de magnésium et évaporée. Le résidu obtenu est purifié par chromatographie sur colonne de silice, élué avec de l'heptane. Après évaporation des solvants, on recueille 7,60 g (60%) du composé attendu, sous la forme d'une poudre blanche de point de fusion 118°C.
1H NMR (CDCl3) d 1,35 (s, 6H), 1,40 (s, 6H), 1,65 à 1,69 (m, 2H), 1,76 à 1,80 (m, 2H), 3,91 (s, 3H), 7,30 (d, 1H, J = 2,0 Hz), 7,34 à 7,46 (m, 3H), 7,59 (d, 2H, J = 7,0 Hz), 7,61 (d, 1H, J = 2.0 Hz).

### (e) (5,5,8,8-tétraméthyl-3-phényl-5,6,7,8-tétrahydro-naphthalène-1-yl)-méthanol.

Dans un tricol de 11, on introduit 80 ml d'éther éthylique et 2,68 g (70,7 mmoles) d'hydrure double de lithium et d'aluminium. Le milieu réactionnel est refroidi à 0°C, puis on introduit goutte à goutte 7,60 g (23,5 mmoles) du composé obtenu à l'exemple 7(d), en solution dans 80 ml d'éther éthylique. Le milieu réactionnel est agité pendant seize heures à la température ambiante, puis on ajoute goutte à goutte une solution saturée de chlorure de sodium, filtre sur Célite(, ajoute de l'eau et de l'éther éthylique. Le produit est extrait par de l'éther éthylique, la phase organique lavée à l'eau jusqu'à pH neutre, séchée sur sulfate de magnésium, filtrée, et les solvants évaporés. On recueille 6,82 g (98%) du composé attendu, sous la forme de cristaux blancs de point de fusion 80-82°C.
1H NMR (CDCl3) d 1,36 (s, 6H), 1,45 (s, 6H), 1,61 (t, 1H, J = 5,8 Hz), 1,71 (s, 4H), 4,95 (d, 2H, J = 5,7 Hz), 7,30 à 7,36 (m, 1H), 7,43 (t, 2H, J = 7,7 Hz), 7,53 (d, 1H, J = 2,1 Hz), 7,58 à 7,61 (m, 3H).

### (f) 5,5,8,8-tétraméthyl-3-phényl-5,6,7,8-tétrahydro-naphthalène-1-carboxaldéhyde.

Dans un ballon de un litre, on mélange 6,56 g (22,2 mmoles) du composé obtenu à l'exemple 7(e), 38,73 g (445,6 mmoles) d'oxyde de manganèse et 500 ml de dichlorométhane. Le milieu réactionnel est agité pendant vingt heures à la température ambiante, puis on filtre l'oxyde de manganèse et lave par du dichlorométhane. Après évaporation des solvants, on recueille 4,44 g (68%) du composé attendu, sous la forme d'une poudre jaune de point de fusion 113°C.
1H NMR (CDCl3) d 1,37 (s, 6H), 1,57 (s, 6H), 1,75 (s, 4H), 7,33 à 7,48 (m, 3H), 7,58 à 7,62 (m, 2H), 7,77 (d, 1H, J = 2,2 Hz), 7,95 (d, 1H, J = 2,2 Hz).

### (g) 1-(5,5,8,8-tétraméthyl-3-phényl-5,6,7,8-tétrahydro-naphthalèn-1-yl)-3-triméthylsilanyl-prop-2-yn-1-ol.

Dans un tricol, on introduit 2,43 ml (17,2 mmoles) de triméthylsilylacétylène et 25 ml de THF. A -78°C et sous courant d'azote, on ajoute goutte à goutte une solution composée de 6,89 ml (17,2 mmoles) de n-butyllithium (2,5 M dans l'hexane), et laisse revenir à la température ambiante. Cette solution est introduite goutte à goutte dans une solution froide (-78°C), composée de 4,20 g (14,4 mmoles) du composé obtenu à l'exemple 7(f) et de 25 ml de THF. On laisse le milieu réactionnel revenir à la température ambiante, verse sur une solution aqueuse de chlorure d'ammonium, extrait avec de l'éther éthylique, décante la phase organique, sèche sur sulfate de magnésium, évapore. On obtient 5,60 g (100%) du composé attendu, sous la forme d'une poudre blanche de point de fusion 145°C.
1H NMR (CDCl3) d 1,34 (s, 3H), 1,36 (s, 3H), 1,48 (s, 3H), 1,51 (s, 3H), 1,66 à 1,76 (m, 4H), 2,19 (br s, 1H), 6,13 (s, 1H), 7,30 à 7,36 (m, 1H), 7,41 à 7,47 (m, 2H), 7,55 (d, 1H, J = 2,0 Hz), 7,60 (d, 2H, J = 7,1 Hz), 7,90 (d, 1H, J = 2,1 Hz).

### (h) 1-(5,5,8,8-tétraméthyl-3-phényl-5,6,7,8-tétrahydro-naphthalèn-1-yl)-prop-2-yn-1-ol.

On mélange dans un tricol de 500 ml, 5,60 g (14,3 mmoles) du composé obtenu à l'exemple 7(g) avec 30 ml de THF, et on ajoute goutte à goutte 15,8 ml (17,4 mmoles) d'une solution de fluorure de tétrabutylammonium (1,1 M dans le THF). On agite à la température ambiante pendant une heure, verse le milieu réactionnel dans l'eau, extrait avec de l'éther éthylique, décante la phase organique, sèche sur sulfate de magnésium, évapore. On recueille 4,07 g (89%) du composé attendu, sous la forme d'une huile jaune.
1H NMR (CDCl3) d 1,34 (s, 3H), 1,35 (s, 3H), 1,48 (s, 3H), 1,52 (s, 3H), 1,66 à 1,75 (m, 4H), 2,30 (br s, 1H), 2,59 (d, 1H, J = 2,1 Hz), 6,16 (d, 1H, J = 2,0 Hz), 7,31 à 7,37 (m, 1H), 7,41 à 7,47 (m, 2H), 7,55 (d, 1H, J = 2,1 Hz), 7,60 (d, 2H, J = 7,1 Hz), 7,88 (d, 1H, J = 2,1 Hz).

### (i) 4-[3-hydroxy-3-(5,5,8,8-tétraméthyl-3-phényl-5,6,7,8-tétrahydro-naphthalèn-1-yl)-prop-1-ynyl]-benzoate d'éthyle.

De manière analogue à l'exemple 3(c), par réaction de 4,07 g (12,8 mmoles) du composé obtenu à l'exemple 7(h) avec 3,53 g (12,8 mmoles) de 4-iodobenzoate d'éthyle, on obtient 4,57 g (77%) de 4-[3-hydroxy-3-(5,5,8,8-tétraméthyl-3-phényl-5,6,7,8-tétrahydro-naphthalèn-1-yl)-prop-1-ynyl]-benzoate d'éthyle, sous la forme d'une poudre orange de point de fusion 121°C.
1H NMR (CDCl3) d 1,35 (s, 3H), 1,35 (t, 3H, J = 7,1 Hz), 1,37 (s, 3H), 1,53 (s, 3H), 1,56 (s, 3H), 1,67 à 1,80 (m, 4H), 2,45 (d, 1H, J = 4,9 Hz), 4,35 (q, 2H, J = 7,1 Hz), 6,40 (d, 1H, J = 4,9 Hz), 7,30 à 7,36 (m, 1H), 7,41 à 7,49 (m, 4H), 7,57 (d, 1H, J = 2,0 Hz), 7,61 (d, 2H, J = 7,1 Hz), 7,95 (d, 1H, J = 2,0 Hz), 7,96 (d, 2H, J = 6,0 Hz).

### EXEMPLE 8

### Acide 4-[3-hydroxy-3-(5,5,8,8-tétraméthyl-3-phényl-5,6,7,8-tétrahydro-naphthalèn-1-yl)-prop-1-ynyl]-benzoïque.

De manière analogue à l'exemple 2, à partir de 3,60 g (7,7 mmoles) du composé obtenu à l'exemple 7(i), on obtient 3,32 g (98%) d'acide 4-[3-hydroxy-3-(5,5,8,8-tétraméthyl-3-phényl-5,6,7,8-tétrahydro-naphthalèn-1-yl)-prop-1-ynyl]-benzoïque, sous la forme d'une poudre beige-orangée de point de fusion 250°C.
1H NMR (DMSO D6) d 1,12 (s, 3H), 1,13 (s, 3H), 1,26 (s, 3H), 1,27 (s, 3H), 1,43 à 1,55 (m, 4H), 5,97 (br s, 1H), 7,13 à 7,19 (m, 1H), 7,25 à 7,33 (m, 4H), 7,36 (d, 1H, J = 1,9 Hz), 7,45 (d, 2H, J = 7,3 Hz), 7,68 (d, 1H, J = 2,0 Hz), 7,70 (d, 2H, J = 8,4 Hz), 12,92 (br s, 1H).

### EXEMPLE 9

### Acide 4-[3-(5,5,8,8-tetraméthyl-3-phényl-5,6,7,8-tétrahydro-naphthalèn-1-yl)-prop-1-ynyl]-benzoïque.

Dans un ballon d'un litre et sous atmosphère d'azote, on introduit 2,00 g (4,6 mmoles) du composé obtenu à l'exemple 8, 1,45 ml (9,1 mmoles) de triéthylsilane, 30 ml de dichlorométhane et 3,5 ml d'acide trifluoroacétique. Le milieu réactionnel est agité pendant deux heures à la température ambiante, hydrolysé à l'aide d'une solution d'HCl 1N, et le produit extrait par de l'éther éthylique. La phase organique est lavée à l'eau, séchée sur sulfate de magnésium et le solvant évaporé à sec. Le résidu obtenu est purifié par chromatographie sur colonne de silice, élué avec un mélange composé de 50% d'acétate d'éthyle et 50% d'heptane. Après évaporation des solvants, on recueille 370 mg (19%) d'acide 4-[3-(5,5,8,8-tétraméthyl-3-phényl-5,6,7,8-tétrahydro-naphthalèn-1-yl)-prop-1-ynyl]-benzoïque, sous la forme d'une poudre blanche de point de fusion 228°C.
1H NMR (DMSO D6) d 1,22 (s, 6H), 1,35 (s, 6H), 4,00 (s, 2H), 7,24 à 7,27 (m, 2H), 7,33 à 7,41 (m, 4H), 7,46 (s, 1H), 7,53 (d, 2H, J = 7,3 Hz), 7,78 (d, 2H, J = 8,2 Hz).

### EXEMPLE 10

### 4-[3-(4,4-diméthyl-thiochroman-5-yl)-3-hydroxy-prop-1-ynyl]-benzoate d'éthyle.

### (a) 1-méthoxy-3-(3-méthyl-but-2-ènylsulfanyl)-benzène.

Dans un ballon de un litre et sous atmosphère d'azote, on introduit 50,45 g (360,0 mmoles) de 3-méthoxythiophénol, 360 ml d'acétone, 14,40 g (360,0 mmoles) de soude en pastilles, et chauffe au reflux pendant trois heures. On ajoute goutte à goutte une solution composée de 53,65 g (360,0 mmoles) de 2-méthyl-4-bromo-2-butène et de 60 ml d'acétone. Le reflux est maintenu pendant seize heures et le milieu réactionnel évaporé à sec. On ajoute de l'eau, extrait à l'acétate d'éthyle, la phase organique est lavée à l'eau, puis à l'aide d'une solution saturée de chlorure de sodium, séchée sur sulfate de magnésium, filtrée et les solvants évaporés. Le résidu obtenu est distillé sous pression réduite (5.10-2 bar / 113°C), pour obtenir 67,81 g (90%) du composé attendu, sous la forme d'une huile jaune pâle.
1H NMR (CDCl3) d 1,62 (s, 3H), 1,72 (s, 3H), 3,55 (d, 2H, J = 7,7 Hz), 3,79 (s, 3H), 5,31 (tt, 1H, J = 7,7 / 1,3 Hz), 6,71 (dt, 1H, J = 8,3 / 1,8 Hz), 6,87 à 6,92 (m, 2H), 7,18 (t, 1H, J = 7,9 Hz).

### (b) 5-méthoxy-4,4-diméthyl-thiochromane.

Dans un ballon, on introduit 62,00 g (298,0 mmoles) du composé obtenu à l'exemple 10(a), 85,00 g (446,0 mmoles) d'acide paratoluènesulfonique et 500 ml de toluène. Le milieu est chauffé à reflux pendant deux heures, refroidi, puis on ajoute de l'eau et de l'acétate d'éthyle et extrait par de l'acétate d'éthyle. On décante la phase organique, lave à l'eau puis à l'aide d'une solution saturée de chlorure de sodium, sèche sur sulfate de magnésium, évapore les solvants. On obtient 65,19 g d'une huile jaune que l'on distille sous pression réduite (5.10-2 bar / 120-122°C), pour obtenir 17,40 g (28%) du composé attendu, sous la forme d'une huile incolore.
1H NMR (CDCl3) d 1,41 (s, 3H), 2,00 à 2,05 (m, 2H), 2,86 à 2,90 (m, 2H), 3,80 (s, 3H), 6,58 (d, 1H, J = 8,1 Hz), 6,72 (dd, 1H, J = 7,9 / 1,2 Hz), 6,98 (t, 1H, J = 8,0 Hz).

### (c) 4,4-diméthyl-thiochroman-5-ol.

Dans un ballon, on introduit 17,40 g (83,5 mmoles) du composé obtenu à l'exemple 10(b), 28,10 g (333,0 mmoles) d'éthanethiolate de sodium et 100 ml de DMF. Le milieu est chauffé à 150°C pendant deux heures, puis agité pendant seize heures à la température ambiante, versé sur un mélange HCI 1N / éther éthylique et extrait par de l'éther éthylique. On décante la phase organique, sèche sur sulfate de magnésium, évapore. Le résidu obtenu est purifié par chromatographie sur colonne de silice, élué avec un mélange composé de 20% d'acétate d'éthyle et de 80% d'heptane. On recueille 14,07 g (87%) du composé attendu, sous la forme d'un solide jaune clair de point de fusion 48°C.
1H NMR (CDCl3) d 1,45 (s, 3H), 2,01 à 2,07 (m, 2H), 2,86 à 2,91 (m, 2H), 5,00 (s, 1H), 6,34 (dd, 1H, J = 7,8 / 1,3 Hz), 6,69 (dd, 1H, J = 7,9 / 1,2 Hz), 6,84 (d, 1H, J = 7,8 Hz).

### (d) trifluoro-méthanesulfonate de 4,4-diméthyl-thiochroman-5-yl.

Dans un ballon de 500 ml, et sous courant d'azote, on introduit 13,63 g (70,1 mmoles) de 4,4-diméthyl-thiochroman-5-ol obtenu à l'exemple 10(c), 11,14 g (91,2 mmoles) de N,N-diméthylaminopyridine et 100 ml de dichlorométhane. On refroidit à 0°C et ajoute goutte à goutte 14,16 ml (84,2 mmoles) d'anhydride triflique. Le milieu réactionnel est agité pendant trente minutes à la température ambiante, puis on ajoute une solution d'HCl 1N et du dichlorométhane. Le produit est extrait par du dichlorométhane, la phase organique lavée à l'eau jusqu'à pH neutre, séchée sur sulfate de magnésium et filtrée. Le résidu obtenu est purifié par chromatographie sur colonne de silice, élué par un mélange composé de 90% d'heptane et 10% d'acétate d'éthyle. Après évaporation des solvants, on recueille 16,32 g (71%) du composé attendu, sous la forme d'une huile jaune.
1H NMR (CDCl3) d 1,46 (s, 3H), 2,01 à 2,06 (m, 2H), 2,93 à 2,98 (m, 2H), 7,00 à 7,12 (m, 3H).

### (e) 4,4-diméthyl-thiochroman-5-carboxylate de méthyle.

De manière analogue à l'exemple 7(d), à partir de 14,23 g (43,6 mmoles) du composé obtenu à l'exemple 10(d), on obtient 8,81 g (85%) du composé attendu, sous la forme d'une huile jaune.
1H NMR (CDCl3) d 1,39 (s, 3H), 1,91 (t, 2H, J = 6,1 Hz), 3,05 (t, 2H, J = 6,1 Hz), 3,89 (s, 3H), 6,96 (dd, 1H, J = 7,4/1,8 Hz), 7,03 (t, 1H, J = 7,4 Hz), 7,16 (dd, 1H, J = 7,5 /1,8 Hz).

### (f) (4,4-diméthyl-thiochroman-5-yl)-méthanol.

Dans un ballon de 500 ml, on mélange 8,81 g (37,3 mmoles) de l'ester obtenu à l'exemple 10(e) et 300 ml de toluène. Après refroidissement à -78°C, on coule goutte à goutte une solution (1M dans le toluène) d'hydrure de diisobutylaluminium en maintenant la température à -78°C. Le milieu réactionnel est agité pendant une heure à cette température, puis on ajoute une pâte aqueuse de sulfate de magnésium, agite et extrait par de l'éther éthylique. La phase organique est séchée sur sulfate de magnésium, filtrée et les solvants évaporés. Le résidu obtenu est purifié par chromatographie sur colonne de silice, élué par un mélange composé de 70% d'heptane et 30% d'acétate d'éthyle. Après évaporation des solvants, on recueille 4,37 g (56%) du composé attendu, sous la forme d'une poudre jaune clair de point de fusion 53°C.
1H NMR (CDCl3) d 1,45 (s, 6H), 2,04 (t, 2H, J = 6,4 Hz), 2,95 (t, 2H, J = 6,4 Hz), 4,87 (d, 2H, J = 5,8 Hz), 7,01 à 7,08 (m, 2H), 7,17 à 7,22 (m, 1H).

### (g) 4,4-diméthyl-thiochroman-5-carboxaldéhyde.

Dans un ballon de 500 ml, on mélange 4,37 g (21,0 mmoles) de l'alcool obtenu à l'exemple 10(f), 36,47 g (419,5 mmoles) d'oxyde de manganèse et 300 ml de dichlorométhane. Le milieu réactionnel est agité pendant vingt heures à la température ambiante, puis on filtre l'oxyde de manganèse sur Celite( et évapore le dichlorométhane. Après évaporation des solvants, on recueille 3,25 g (75%) du composé attendu, sous la forme d'une huile jaune.
1H NMR (CDCl3) d 1,54 (s, 6H), 2,03 (t, 2H, J = 5,9 Hz), 3,01 (t, 2H, J = 6,0 Hz), 7,15 (d, 1H, J = 7,6 Hz), 7,25 à 7,29 (m, 1H), 7,49 (dd, 1H, J = 7,4 / 1,4 Hz), 10,73 (s, 1H).

### (h) 1-(4,4-diméthyl-thiochroman-5-yl)-3-triméthylsilanyl-prop-2-yn-1-ol.

De manière analogue à l'exemple 7(g), à partir de 3,25 g (15,7 mmoles) du composé obtenu à l'exemple 10(g), on obtient 4,79 g (100%) de 1- (4,4-diméthyl-thiochroman-5-yl)-3-triméthylsilanyl-prop-2-yn-1-ol, sous la forme d'une huile jaune.
1H NMR (CDCl3) d 0,16 (s, 9H), 1,48 (s, 3H), 1,50 (s, 3H), 2,04 à 2,09 (m, 2H), 2,14 (d, 1H, J = 5,2 Hz), 2,87 à 2,93 (m, 2H), 6,04 (d, 1H, J = 5,1 Hz), 7,05 à 7,13 (m, 2H), 7,50 à 7,54 (q, 1H, J = 3,1 Hz).

### (i) 1-(4,4-diméthyl-thiochroman-5-yl)-prop-2-yn-1-ol.

De manière analogue à l'exemple 7(h), à partir de 4,79 g (15,7 mmoles) du composé obtenu à l'exemple 10(h), on obtient 3,34 g (89%) de 1-(4,4-diméthyl-thiochroman-5-yl)-prop-2-yn-1-ol, sous la forme de cristaux beiges de point de fusion 88°C.
1H NMR (CDCl3) d 1,49 (s, 3H), 1,51 (s, 3H), 2,04 à 2,10 (m, 2H), 2,26 (d, 1H, J = 5,1 Hz), 2,59 (d, 1H, J = 2,2 Hz), 2,88 à 2,94 (m, 2H), 6,07 (br s, 1H), 7,07 à 7,14 (m, 2H), 7,52 à 7,55 (q, 1H, J = 3,0 Hz).

### (j) 4-[3-(4,4-diméthyl-thiochroman-5-yl)-3-hydroxy-prop-1-ynyl]-benzoate d'éthyle.

De manière analogue à l'exemple 3(c), par réaction de 3,34 g (14,4 mmoles) du composé obtenu à l'exemple 10(i) avec 3,97 g (14,4 mmoles) de 4-iodobenzoate d'éthyle, on obtient 4,66 g (85%) de 4-[3-(4,4-diméthyl-thiochroman-5-yl)-3-hydroxy-prop-1-ynyl]-benzoate d'éthyle, sous la forme d'une poudre orangée de point de fusion 108°C.
1H NMR (CDCl3) d 1,39 (t, 3H, J = 7,1 Hz), 1,54 (s, 3H), 1,56 (s, 3H), 2,08 à 2,13 (m, 2H), 2,28 (d, 1H, J = 5,3 Hz), 2,90 à 2,96 (m, 2H), 4,37 (q, 2H, J = 7,1 Hz), 6,31 (d, 1H, J = 5,3 Hz), 7,09 à 7,17 (m, 2H), 7,48 (d, 2H, J = 8,3 Hz), 7,60 (dd, 1H, J = 6,4 / 2,8 Hz), 7,98 (d, 2H, J = 8,4 Hz).

### EXEMPLE 11

### Acide 4-[3-(4,4-diméthvl-thiochroman-5-yl)-3-hydroxy-prop-1-ynyl]-benzoïque.

De manière analogue à l'exemple 2, à partir de 4,66 g (12,3 mmoles) du composé obtenu à l'exemple 10(j), on obtient 3,41 g (78%) d'acide 4-[3-(4,4-diméthyl-thiochroman-5-yl)-3-hydroxy-prop-1-ynyl]-benzoïque, sous la forme d'un solide marron de point de fusion 198°C.
1H NMR (CDCl3) d 1,54 (s, 3H), 1,56 (s, 3H), 2,08 à 2,12 (m, 2H), 2,90 à 2,94 (m, 2H), 6,29 (s, 1H), 7,10 à 7,16 (m, 2H), 7,48 (d, 2H, J = 8,2 Hz), 7,61 (dd, 1H, J = 6,7 / 2,3 Hz), 7,99 (d, 2H, J = 8,3 Hz).

### EXEMPLE 12

### Acide 4-[3-(4,4-diméthyl-thiochroman-5-yl)-prop-1-ynyl]-benzoïque.

De manière analogue à l'exemple 9, à partir de 2,06 g (5,82 mmoles) du composé obtenu à l'exemple 11, on obtient 1,00 g (51%) d'acide 4-[3-(4,4-diméthyl-thiochroman-5-yl)-prop-1-ynyl]-benzoïque, sous la forme de cristaux beiges de point de fusion 207 °C.
1H NMR (DMSO D6) d 1,51 (s, 6H), 2,05 à 2,10 (m, 2H), 2,92 à 2,97 (m, 2H), 4,01 (s, 2H), 7,01 7,03 (m, 2H), 7,25 à 7,31 (m, 1H), 7,45 (d, 2H, J = 8,3 Hz), 7,97 (d, 2H, J = 8,3 Hz).

### EXEMPLE 13

### 4-[3-(3,5-di-tert-butyl-2-méthoxyméthoxy-phényl)-3-hydroxy-prop-1-ynyl]-benzoate d'éthyle.

### (a) 1-bromo-3,5-di-tert-butyl-2-méthoxyméthoxy-benzène.

Dans un tricol de un litre, on introduit 40,00 g (140,2 mmoles) de 2,4-di-tert.butyl-6-bromophénol et 400 ml de DMF. On refroidit la solution obtenue à 5-10°C, ajoute 4,70 g de d'hydrure de sodium et agite à 10°C pendant trente minutes. On ajoute alors goutte à goutte 11,7 ml (154,0 mmoles) d'éther méthylique de chlorométhyle et agite le milieu réactionnel pendant une heure à la température ambiante. On verse le milieu réactionnel dans un mélange HCI 1N / éther éthylique, extrait par de l'éther éthylique, décante la phase organique, sèche sur sulfate de magnésium, évapore. On recueille 46,00 g (100%) du composé attendu, sous la forme d'une huile orange.
1H NMR (CDCl3) d 1,28 (s, 9H), 1,43 (s, 9H), 3,69 (s, 3H), 5,21 (s, 2H), 7,30 (d, 1H, J = 2,4 Hz), 7,39 (d, 1H, J = 2,4 Hz).

### (b) 3,5-di-tert-butyl-2-méthoxyméthoxy-benzaldéhyde.

Dans un tricol et sous courant d'azote, on introduit 46,00 g (140,0 mmoles) du composé obtenu à l'exemple 13(a) et 500 ml de THF. On ajoute goutte à goutte à -78°C, 61,5 ml (154,0 mmoles) d'une solution de n.butyllithium (2,5M dans l'hexane) et agite trente minutes à cette même température. On ajoute ensuite goutte à goutte 13,0 ml (168,0 mmoles) de DMF, et laisse remonter à la température ambiante. On acidifie le milieu réactionnel avec de l'acide chlorhydrique (1N), extrait avec de l'éther éthylique, décante la phase organique, sèche sur sulfate de magnésium, évapore. On recueille 46,00 g (100%) du composé attendu, sous la forme d'une huile orange.
1H NMR (CDCl3) d 1,32 (s, 9H), 1,44 (s, 9H), 3,64 (s, 3H), 5,02 (s, 2H), 7,64 (d, 1H, J = 2,6 Hz), 7,72 (d, 1H, J = 2,6 Hz), 10,22 (s, 1H).

### (c) 1-(3,5-di-tert-butyl-2-méthoxyméthoxy-phényl)-3-triméthylsilanyl-prop-2-yn-1-ol.

Dans un tricol et sous courant d'azote, on introduit 18,60 g (190,0 mmoles) de triméthylsilylacétylène, 190 ml de THF et refroidit la solution ainsi obtenue à -78°C. On ajoute goutte à goutte à -70°C, 76,0 ml (190,0 mmoles) d'une solution de n.butyllithium (2,5M dans l'hexane), agite trente minutes à cette même température et remonte à -20°C. On coule goutte à goutte cette solution sur une solution froide (-70°C), composée de 44,00 g (158,0 mmoles) du composé obtenu à l'exemple 13(b) en solution dans 550 ml de THF anhydre. La température du milieu réactionnel est remontée à la température ambiante en deux heures, puis on acidifie le milieu réactionnel avec de l'acide chlorhydrique (1N), extrait avec de l'éther éthylique, décante la phase organique, sèche sur sulfate de magnésium, évapore. On recueille 59,00 g (100%) du composé attendu, sous la forme d'une huile orange.
1H NMR (CDCl3) d 1,12 (s, 9H), 1,18 (s, 9H), 3,49 (s, 3H), 3,81 (d, 1H, J = 5,4 Hz), 4,68 (d, 1H, J = 6,3 Hz), 4,88 (d, 1H, J = 6,3 Hz), 5,55 (d, 1H, J = 5,3 Hz), 7,16 (d, 1H, J = 2,5 Hz), 7,56 (d, 1H, J = 2,5 Hz).

### (d) 1-(3,5-di-tert-butyl-2-méthoxyméthoxy-phényl)-prop-2-yn-1-ol.

Dans un tricol et sous courant d'azote, on introduit 58,00 g (154,0 mmoles) du composé obtenu à l'exemple 13(c), 300 ml de THF et coule goutte à goutte une solution (1M dans le THF) de fluorure de tétrabutylammonium. On agite pendant deux heures à la température ambiante, puis on acidifie le milieu réactionnel avec de l'acide chlorhydrique (1N), extrait avec de l'éther éthylique, décante la phase organique, sèche sur sulfate de magnésium, évapore. On recueille 6,20 g (13%) du composé attendu, sous la forme d'une huile orange et 12,00 g (30%) de 1-(3,5-di-tert-butyl-2-hydroxy-phényl)-prop-2-yn-1-ol, sous la forme d'une huile orange.
1H NMR du composé attendu (CDCl3) d 1,32 (s, 9H), 1,39 (s, 9H), 2,61 (d, 1H, J = 2,2 Hz), 3,70 (s, 3H), 3,90 (d, 1H, J = 5,5 Hz), 4,90 (d, 1H, J = 6,3 Hz), 5,08 (d, 1H, J = 6,2 Hz), 5,79 (dd, 1H, J = 5,4 / 2,3 Hz), 7,37 (d, 1H, J = 2,5 Hz), 7,70 (d, 1H, J = 2,5 Hz).
1H NMR du 1-(3,5-di-tert-butyl-2-hydroxy-phényl)-prop-2-yn-1-ol (CDCl3) d 1,30 (s, 9H), 1,43 (s, 9H), 2,72 (br s, 1H), 2,80 (d, 1H, J = 2,3 Hz), 4,94 à 5,05 (m, 1H), 5,66 (br s, 1H), 7,27 (d, 1H, J = 2,3 Hz), 7,32 (d, 1H, J = 2,4 Hz).

### (e) 4-[3-(3,5-di-tert-butyl-2-méthoxyméthoxy-phényl)-3-hydroxy-prop-1-ynyl]-benzoate d'éthyle.

Dans un ballon, on introduit successivement 6,00 g (19,7 mmoles) du composé obtenu à l'exemple 13(d), 5,40 g (19,7 mmoles) de 4-iodobenzoate d'éthyle et 40 ml de triéthylamine. Le milieu réactionnel est dégazé à l'azote pendant vingt minutes, puis on ajoute 375 mg de CuI, et 700 mg de chlorure de bis(triphénylphosphine)palladium(II). On agite à la température ambiante pendant cinq heures, verse le milieu réactionnel dans l'eau, acidifie par de l'acide chlorhydrique 1N, extrait par de l'éther éthylique, décante la phase organique, sèche sur sulfate de magnésium, évapore. Le résidu obtenu est purifié par chromatographie sur colonne de silice, élué par un mélange composé de 10% d'acétate d'éthyle et de 90% d'heptane. Après évaporation des solvants, on recueille 6,00 g (69%) de 4-[3-(3,5-di-tert-butyl-2-méthoxyméthoxy-phényl)-3-hydroxy-prop-1-ynyl]-benzoate d'éthyle, sous la forme d'une poudre orangée de point de fusion 89-91°C.
1H NMR (CDCl3) d 1,34 (s, 9H), 1,39 (t, 3H, J = 7,1 Hz), 1,41 (s, 9H), 3,73 (s, 3H), 4,09 (d, 1H, J = 5,5 Hz), 4,37 (q, 2H, J = 7,1 Hz), 4,93 (d, 1H, J = 6,3 Hz), 5,12 (d, 1H, J = 6,3 Hz), 6,00 (d, 1H, J = 5,5 Hz), 7,40 (d, 1H, J = 2,5 Hz), 7,53 (d, 2H, J = 8,4 Hz), 7,79 (d, 1H, J = 2,5 Hz), 7,99 (d, 2H, J = 8,4 Hz).

### EXEMPLE 14

### Acide 4-[3-(3,5-di-tert-butyl-2-méthoxyméthoxy-phényl)-3-hydroxy-prop-1-ynyl]-benzoïque.

De manière analogue à l'exemple 2, à partir de 1,50 g (3,3 mmoles) du composé obtenu à l'exemple 13(e), on obtient 1,20 g (85%) d'acide 4-[3-(3,5-di-tert-butyl-2-méthoxyméthoxy-phényl)-3-hydroxy-prop-1-ynyl]-benzoique, sous la forme d'une poudre beige de point de fusion 197°C.
1H NMR (CDCl3) d 1,33 (s, 9H), 1,41 (s, 9H), 3,73 (s, 3H), 4,35 (br s, 1H), 4,97 (d, 1H, J = 6,1 Hz), 5,12 (d, 1H, J = 6,1 Hz), 6,00 (s, 1H), 7,38 (d, 1H, J = 2,5 Hz), 7,51 (d, 2H, J = 8,4 Hz), 7,78 (d, 1H, J = 2,5 Hz), 8,00 (d, 2H, J = 8,4 Hz).

### EXEMPLE 15

### 4-[3-(3,5-di-tert-butyl-2-hydroxy-phényl)-3-hydroxy-prop-1-ynyl]-benzoate d'éthyle.

De manière analogue à l'exemple 13(e), par réaction de 10,00 g (38,4 mmoles) de 1-(3,5-di-tert-butyl-2-hydroxy-phényl)-prop-2-yn-1-ol obtenu à l'exemple 13(d) avec 10,60 g (38,4 mmoles) de 4-iodobenzoate d'éthyle, on obtient 5,00 g (32%) de 4-[3-(3,5-di-tert-butyl-2-hydroxy-phényl)-3-hydroxy-prop-1-ynyl]-benzoate d'éthyle, sous la forme d'un solide blanc cassé de point de fusion 142-144°C.
1H NMR (CDCl3) d 1,31 (s, 9H), 1,39 (t, 3H, J = 7,1 Hz), 1,44 (s, 9H), 2,80 (d, 1H, J = 6,2 Hz), 4,37 (q, 2H, J = 7,1 Hz), 5,90 (d, 1H, J = 6,2 Hz), 7,34 (s, 2H), 7,37 (s, 1H), 7,53 (d, 2H, J = 8,4 Hz), 8,02 (d, 2H, J = 8,4 Hz).

### EXEMPLE 16

### 4-[3-(3,5-di-tert-butyl-2-hydroxy-phényl)-prop-1-ynyl]-benzoate d'éthyle.

De manière analogue à l'exemple 9, à partir de 3,00 g (7,3 mmoles) du composé obtenu à l'exemple 15, on obtient 1,30 g (45%) de 4-[3-(3,5-di-tert-butyl-2-hydroxy-phényl)-prop-1-ynyl]-benzoate d'éthyle, sous la forme d'une poudre blanche de point de fusion 113-115°C.
1H NMR (CDCl3) d 1,31 (s, 9H), 1,39 (t, 3H, J = 7,1 Hz), 1,44 (s, 9H), 3,80 (s, 2H), 4,37 (q, 2H, J = 7,1 Hz), 6,77 (br s, 1H), 7,13 (d, 1H, J = 2,3 Hz), 7,27 (d, 1H, J = 2,3 Hz), 7,48 (d, 2H, J = 8,3 Hz), 7,98 (d, 2H, J = 8,4 Hz).

### EXEMPLE 17

### 4-[3-(3,5-di-tert-butyl-2-méthoxy-phényl)-3-hydroxy-prop-1-ynyl]-benzoate d'éthyle.

### (a) 1-bromo-3,5-di-tert-butyl-2-méthoxy-benzène.

De manière analogue à l'exemple 13(a), à partir de 25,00 g (87,6 mmoles) de 2,4-di-tert.butyl-6-bromophénol et de 13,70 g (96,4 mmoles) d'iodure de méthyle, on obtient 27,00 g (100%) de 1-bromo-3,5-di-tert-butyl-2-méthoxy-benzène, sous la forme d'une huile orange.
1H NMR (CDCl3) d 1,29 (s, 9H), 1,39 (s, 9H), 3,91 (s, 3H), 7,27 (d, 1H, J = 2,4 Hz), 7,40 (d, 1H, J = 2,4 Hz).

### (b) 3,5-di-tert-butyl-2-méthoxy-benzaldéhyde.

De manière analogue à l'exemple 13(b), à partir de 25,00 g (83,5 mmoles) du composé obtenu à l'exemple 17(a), on obtient 21,00 g (100%) du composé attendu, sous la forme d'une huile orange.
1H NMR (CDCl3) d 1,32 (s, 9H), 1,43 (s, 9H), 3,93 (s, 3H), 7,61 (d, 1H, J = 2,6 Hz), 7,71 (d, 1H, J = 2,5 Hz), 10,34 (s, 1H).

### (c) 1-(3,5-di-tert-butyl-2-méthoxy-phényl)-3-triméthylsilanyl-prop-2-yn-1-ol.

De manière analogue à l'exemple 13(c), à partir de 21,00 g (85,0 mmoles) du composé obtenu à l'exemple 17(b), on obtient 30,00 g (100%) du composé attendu, sous la forme d'une poudre beige de point de fusion 104-106°C.
1H NMR (CDCl3) d 1,13 (s, 9H), 1,20 (s, 9H), 2,39 (d, 1H, J = 4,7 Hz), 3,69 (s, 3H), 5,59 (d, 1H, J = 4,0 Hz), 7,15 (d, 1H, J = 2,5 Hz), 7,43 (d, 1H, J = 2,5 Hz).

### (d) 1-(3,5-di-tert-butyl-2-méthoxy-phényl)-prop-2-yn-1-ol.

De manière analogue à l'exemple 13(d), à partir de 23,00 g (66,0 mmoles) du composé obtenu à l'exemple 17(c), on obtient 25,00 g (100%) du composé attendu, sous la forme d'une huile orange.
1H NMR (CDCl3) d 1,32 (s, 9H), 1,40 (s, 9H), 2,63 (d, 1H, J = 2,2 Hz), 3,88 (s, 3H), 5,81 (d, 1H, J = 2,2 Hz), 7,35 (d, 1H, J = 2,5 Hz), 7,58 (d, 1H, J = 2,5 Hz).

### (e) 4-[3-(3,5-di-tert-butyl-2-méthoxy-phényl)-3-hydroxy-prop-1-ynyl]-benzoate d'éthyle.

De manière analogue à l'exemple 13(e), par réaction de 23,30 g (85,0 mmoles) du composé obtenu à l'exemple 17(d), avec 23,50 g (85,0 mmoles) de 4-iodobenzoate d'éthyle, on obtient 20,00 g (55%) de 4-[3-(3,5-di-tert-butyl-2-méthoxy-phényl)-3-hydroxy-prop-1-ynyl]-benzoate d'éthyle, sous la forme d'une poudre grise de point de fusion 101-103°C.
1H NMR (CDCl3) d 1,34 (s, 9H), 1,39 (t, 3H, J = 5,2 Hz), 1,42 (s, 9H), 2,74 (d, 1H, J = 5,4 Hz), 3,93 (s, 3H), 4,37 (q, 2H, J = 7,1 Hz), 6,04 (d, 1H, J = 5,4 Hz), 7,37 (d, 1H, J = 2,5 Hz), 7,50 (d, 2H, J = 8,4 Hz), 7,65 (d, 1H, J = 2,5 Hz), 7,99 (d, 2H, J = 8,4 Hz).

### EXEMPLE 18

### Acide 4-[3-(3,5-di-tert-butyl-2-méthoxy-phényl)-3-hydroxy-prop-1-ynyl]-benzoïque.

De manière analogue à l'exemple 2, à partir de 5,00 g (11,8 mmoles) du composé obtenu à l'exemple 17(e), on obtient 4,50 g (96%) d'acide 4-[3-(3,5-di-tert-butyl-2-méthoxy-phényl)-3-hydroxy-prop-1-ynyl]-benzoïque, sous la forme d'un solide jaune clair de point de fusion 208-209°C.
1H NMR (CDCl3) d 1,29 (s, 9H), 1,37 (s, 9H), 3,83 (s, 3H), 5,80 (d, 1H, J = 5,0 Hz), 6,19 (d, 1H, J = 5,6 Hz), 7,27 (d, 1H, J = 2,5 Hz), 7,53 (d, 2H, J = 8,3 Hz), 7,62 (d, 1H, J = 2,4 Hz), 7,93 (d, 2H, J = 8,3 Hz), 13,14 (br s, 1H).

### EXEMPLE 19

### Acide 4- [3-(3,5-di-tert-butyl-2-méthoxy-phényl)-prop-1-ynyl]-benzoïque.

De manière analogue à l'exemple 9, à partir de 1,50 g (3,8 mmoles) du composé obtenu à l'exemple 18, on obtient 1,40 g (97%) d'acide 4-[3-(3,5-di-tert-butyl-2-méthoxy-phényl)-prop-1-ynyl]-benzoique, sous la forme d'une poudre blanche de point de fusion 237-239°C.
1H NMR (CDCl3) d 1,33 (s, 9H), 1,41 (s, 9H), 3,83 (s, 3H), 3,87 (s, 2H), 7,27 (d, 1H, J = 2,2 Hz), 7,46 (d, 1H, J = 2,2 Hz), 7,47 (d, 2H, J = 8,2 Hz), 7,99 (d, 2H, J = 8,2 Hz).

### EXEMPLE 20

### 4-[3-(5-tert-butyl-4-méthoxyméthoxy-biphényl-3-yl)-3-hydroxy-prop-1-ynyl]-benzoate d'éthyle.

### (a) 2-tert-butyl-4-bromophénol.

Dans un tricol de 250 ml, on mélange 80,00 g (426,0 mmoles) de 4-bromophénol et 8,00 g de résine sulfonique acide DOWEX 50WX8. Le mélange est chauffé à 80°C et on fait passer un courant d'isobutylène pendant 30 heures. Le milieu réactionnel est refroidi et le résidu est purifié par passage sur colonne de silice, élué par un mélange composé de 95% de dichlorométhane et de 5% d'heptane. On recueille 88,00 g (90%) du composé attendu sous la forme d'une huile jaune.
1H NMR (CDCl3) d 1,38 (s, 9H), 4,79 (s, 1H), 6,55 (d, 1H, J = 8,4 Hz), 7,16 (dd, 1H, J = 8,4 / 2,4 Hz), 7,35 (d, 1H, J = 2,4 Hz).

### (b) 3-tert-butyl-biphényl-4-ol.

De manière analogue à l'exemple 7(b), par réaction de 40,00 g (175,0 mmoles) du composé obtenu à l'exemple 20(a) avec 34,60 g (283,0 mmoles) d'acide phénylboronique, on obtient 27,00 g (68%) du composé attendu, sous la forme d'une huile brune.
1H NMR (CDCl3) d 1,46 (s, 9H), 4,99 (s, 1H), 6,74 (d, 1H, J = 8,1 Hz), 7,28 (d, 1H, J = 2,3 Hz), 7,31 (d, 1H, J = 2,4 Hz), 7,41 (t, 2H, J = 7,2 Hz), 7,50 (d, 1H, J = 2,2 Hz), 7,53 (s, 1H), 7,56 (d, 1H, J = 1,4 Hz).

### (c) 5-bromo-3-tert-butyl-biphényl-4-ol.

Dans un ballon, on introduit 27,00 g (120,0 mmoles) du composé obtenu à l'exemple 20(b) et 120 ml de dichlorométhane. On refroidit à 0°C, ajoute goutte à goutte 6,4 ml (131,0 mmoles) de brome et agite dix minutes à 0°C. On ajoute une solution saturée de thiosulfate de sodium, extrait par du dichlorométhane, décante la phase organique, lave à l'eau jusqu'à pH neutre, sèche sur sulfate de magnésium, évapore. On recueille 32,00 g (88%) du produit attendu, sous la forme d'une huile jaune.
1H NMR (CDCl3) d 1,45 (s, 9H), 5,83 (s, 1H), 7,28 à 7,34 (m, 1H), 7,40 (d, 2H, J = 7,6 Hz), 7,44 (d, 1H, J = 1,9 Hz), 7,49 à 7,53 (d, 2H, J = 8,6 Hz), 7,57 (d, 1H, J = 2,2 Hz).

### (d) 5-bromo-3-tert-butyl-4-méthoxyméthoxy-biphényle.

De manière analogue à l'exemple 13(a), par réaction de 7,30 g (24,0 mmoles) du composé obtenu à l'exemple 20(c) avec 2,0 ml (26,4 mmoles) d'éther méthylique de chlorométhyle, on obtient 8,00 g (100%) du composé attendu, sous la forme d'une huile orange.
1H NMR (CDCl3) d 1,48 (s, 9H), 3,71 (s, 3H), 5,16 (s, 2H), 7,34 à 7,46 (m, 3H), 7,51 à 7,54 (m, 3H), 7,64 (d, 1H, J = 2,0 Hz).

### (e) 5-tert-butyl-4-méthoxyméthoxy-biphényl-3-carboxaldéhyde.

De manière analogue à l'exemple 3(a), à partir de 7,80 g (23,0 mmoles) du composé obtenu à l'exemple 20(d), on obtient 4,31 g (63%) du composé attendu, sous la forme d'un solide jaune de point de fusion 92-94°C.
1H NMR (CDCl3) d 1,49 (s, 9H), 3,66 (s, 3H), 5,09 (s, 2H), 7,38 (d, 1H, J = 8,5 Hz), 7,44 (t, 2H, J = 7,0 Hz), 7,58 (d, 2H, J = 8,5 Hz), 7,82 (d, 1H, J = 2,5 Hz), 7,94 (d, 1H, J = 2,4 Hz), 10,27 (s, 1H).

### (f) 1-(5-tert-butyl-4-méthoxyméthoxy-biphényl-3-yl)-3-triméthylsilanyl-prop-2-yn-1-ol.

De manière analogue à l'exemple 7(g), à partir de 4,30 g (14,4 mmoles) du composé obtenu à l'exemple 20(e), on obtient 4,00 g (70%) du composé attendu, sous la forme d'un solide jaune de point de fusion 90-91°C.
1H NMR (CDCl3) d 0,21 (s, 9H), 1,45 (s, 9H), 3,74 (s, 3H), 3,87 (d, 1H, J = 5,5 Hz), 4,96 (d, 1H, J = 6,2 Hz), 5,15 (d, 1H, J = 6,2 Hz), 5,84 (d, 1H, J = 5,5 Hz), 7,36 (d, 1H, J = 7,1 Hz), 7,46 (t, 2H, J = 7,0 Hz), 7,59 à 7,62 (m, 3H), 7,96 (d, 1H, J = 2,4 Hz).

### (g) 1-(5-tert-butyl-4-méthoxyméthoxy-biphényl-3-yl)-prop-2-yn-1-ol.

De manière analogue à l'exemple 7(h), à partir de 4,00 g (10,1 mmoles) du composé obtenu à l'exemple 20(f), on obtient 3,27 g (100%) du composé attendu, sous la forme d'une huile orange.
1H NMR (CDCl3) d 1,37 (s, 9H), 2,55 (d, 1H, J = 2,3 Hz), 3,66 (s, 3H), 4,89 (d, 1H, J = 6,3 Hz), 5,07 (d, 1H, J = 6,2 Hz), 5,79 (d, 1H, J = 2,1 Hz), 7,24 à 7,46 (m, 3H), 7,50 à 7,54 (m, 3H), 7,85 (d, 1H, J = 2,3 Hz).

### (h) 4-[3-(5-tert-butyl-4-méthoxyméthoxy-biphényl-3-yl)-3-hydroxy-prop-1-ynyl]-benzoate d'éthyle.

De manière analogue à l'exemple 3(c), par réaction de 3,20 g (9,9 mmoles) du composé obtenu à l'exemple 20(g) avec 3,00 g (10,8 mmoles) de 4-iodobenzoate d'éthyle, on obtient 3,00 g (65%) de 4-[3-(5-tert-butyl-4-méthoxyméthoxy-biphényl-3-yl)-3-hydroxy-prop-1-ynyl]-benzoate d'éthyle, sous la forme d'une huile brune.
1H NMR (CDCl3) d 1,39 (t, 3H, J = 7,1 Hz), 1,46 (s, 9H), 3,76 (s, 3H), 3,99 (d, 1H, J = 5,5 Hz), 4,37 (q, 2H, J = 7,2 Hz), 4,99 (d, 1H, J = 6,3 Hz), 5,17 (d, 1H, J = 6,3 Hz), 7,35 (d, 1H, J = 7,1 Hz), 7,44 (t, 1H, J = 7,6 Hz), 7,53 (d, 2H, J = 8,3 Hz), 7,57 à 7,61 (m, 4H), 7,96 (s, 1H), 7,98 (d, 2H, J = 8,1 Hz).

### EXEMPLE 21

### Acide 4-[3-(5-tert-butyl-4-méthoxyméthoxy-biphényl-3-yl)-3-hydroxy-prop-1-ynyl]-benzoïque.

De manière analogue à l'exemple 2, à partir de 1,50 g (3,2 mmoles) du composé obtenu à l'exemple 20(h), on obtient 970 mg (70%) d'acide 4-[3-(5-tert-butyl-4-méthoxyméthoxy-biphényl-3-yl)-3-hydroxy-prop-1-ynyl]-benzoique, sous la forme d'une poudre beige de point de fusion 162-164°C.
1H NMR (DMSO D6) d 1,44 (s, 9H), 3,63 (s, 3H), 5,11 (d, 1H, J = 5,2 Hz), 5,18 (d, 1H, J = 5,2 Hz), 5,94 (d, 1H, J = 6,2 Hz), 6,30 (d, 1H, J = 6,3 Hz), 7,37 (d, 1H, J = 7,3 Hz), 7,46 (d, 2H, J = 7,6 Hz), 7,52 à 7,55 (m, 3H), 7,62 (d, 2H, J = 7,5 Hz), 7,85 (d, 1H, J = 2,0 Hz), 7,91 (d, 2H, J = 8,1 Hz), 13,14 (s, 1H).

### EXEMPLE 22

### 4-[3-(5-tert-butyl-4-méthoxy-biphényl-3-yl)-3-hydroxy-prop-1-ynyl]-benzoate d'éthyle.

### (a) 5-bromo-3-tert-butyl-4-méthoxy-biphényle.

De manière analogue à l'exemple 13(a), par réaction de 4,00 g (13,0 mmoles) du composé obtenu à l'exemple 20(c) avec 890 µl (14,3 mmoles) d'iodure de méthyle, on obtient 4,09 g (98%) du composé attendu, sous la forme d'une huile jaune.
1H NMR (CDCl3) d 1,44 (s, 9H), 3,97 (s, 3H), 7,34 à 7,54 (m, 5H), 7,47 (d, 1H, J = 2,1 Hz), 7,65 (d, 1H, J = 2,0 Hz).

### (b) 5-tert-butyl-4-méthoxy-biphényl-3-carboxaldéhyde.

De manière analogue à l'exemple 3(a), à partir de 3,80 g (12,0 mmoles) du composé obtenu à l'exemple 22(a), on obtient 2,29 g (71%) du composé attendu, sous la forme d'un solide jaune de point de fusion 45°C. 1H NMR (CDCl3) d 1,47 (s, 9H), 3,99 (s, 3H), 7,32 à 7,59 (m, 5H), 7,79 (d, 1H, J = 2,1 Hz), 7,93 (d, 1H, J = 2,2 Hz), 10,40 (s, 1H).

### (c) 1-(5-tert-butyl-4-méthoxy-biphényl-3-yl)-3-triméthylsilanyl-prop-2-yn-1-ol.

De manière analogue à l'exemple 7(g), à partir de 2,29 g (8,5 mmoles) du composé obtenu à l'exemple 22(b), on obtient 2,00 g (64%) du composé attendu, sous la forme d'un solide jaune de point de fusion 94-96°C.
1H NMR (CDCl3) d 0,21 (s, 9H), 1,46 (s, 3H), 2,56 (d, 1H, J = 5,3 Hz), 3,96 (s, 3H), 5,86 (d, 1H, J = 5,2 Hz), 7,37 (d, 1H, J = 7,1 Hz), 7,46 (t, 2H, J = 7,0 Hz), 7,56 (d, 1H, J = 2,4 Hz), 7,60 (d, 2H, J = 7,6 Hz), 7,83 (d, 1H, J = 2,3 Hz).

### (d) 1-(5-tert-butyl-4-méthoxy-biphényl-3-yl)-prop-2-yn-1-ol.

De manière analogue à l'exemple 7(h), à partir de 2,00 g (5,5 mmoles) du composé obtenu à l'exemple 22(c), on obtient 1,52 g (95%) du composé attendu, sous la forme d'une huile jaune.
1H NMR (CDCl3) d 1,38 (s, 9H), 2,57 (d, 1H, J = 2,3 Hz), 3,87 (s, 3H), 5,79 (br s, 1H), 7,24 à 7,40 (m, 6H), 7,74 (d, 1H, J = 2,3 Hz).

### (e) 4-[3-(5-tert-butyl-4-méthoxy-biphényl-3-yl)-3-hydroxy-prop-1-ynyl]-benzoate d'éthyle.

De manière analogue à l'exemple 3(c), par réaction de 1,50 g (5,1 mmoles) du composé obtenu à l'exemple 22(d) avec 1,55 g (5,6 mmoles) de 4-iodobenzoate d'éthyle, on obtient 1,88 g (83%) de 4-[3-(5-tert-butyl-4-méthoxy-biphényl-3-yl)-3-hydroxy-prop-1-ynyl]-benzoate d'éthyle, sous la forme d'une huile rougeâtre.
1H NMR (CDCl3) d 1,39 (t, 3H, J = 7,2 Hz), 1,46 (s, 9H), 2,67 (d, 1H, J = 5,3 Hz), 3,99 (s, 3H), 4,37 (q, 2H, J = 7,1 Hz), 6,10 (d, 1H, J = 5,2 Hz), 7,34 à 7,60 (m, 8H), 7,85 (d, 1H, J = 2,3 Hz), 7,98 (d, 2H, J = 8,4 Hz).

### EXEMPLE 23

### Acide 4-[3-(5-tert-butyl-4-méthoxy-biphényl-3-yl)-3-hydroxy-prop-1-ynyl]-benzoïque.

De manière analogue à l'exemple 2, à partir de 1,88 g (4,2 mmoles) du composé obtenu à l'exemple 22(e), on obtient 1,25 g (72%) d'acide 4-[3-(5-tert-butyl-4-méthoxy-biphényl-3-yl)-3-hydroxy-prop-1-ynyl]-benzoïque, sous la forme d'une poudre beige de point de fusion 165-167°C.
1H NMR (DMSO D6) d 1,42 (s, 9H), 3,90 (s, 3H), 5,88 (br s, 1H), 6,32 (br s, 1H), 7,37 (d, 1H, J = 7,2 Hz), 7,45 à 7,51 (m, 3H), 7,54 (d, 2H, J = 8,3 Hz), 7,62 (d, 2H, J = 7,2 Hz), 7,83 (d, 1H, J = 2,3 Hz), 7,91 (d, 2H, J = 8,3 Hz).

### EXEMPLE 24

### 4-[3-(3,5-di-tert-butyl-2-méthoxy-phényl)-3-méthoxy-prop-1-ynyl]-benzoate d'éthyle.

### (a) 1-(3,5-di-tert-butyl-2-méthoxy-phényl)-1-méthoxy-prop-2-yne.

De manière analogue à l'exemple 13(a), par réaction de 1,30 g (5,0 mmoles) de 1-(3,5-di-tert-butyl-2-hydroxy-phényl)-prop-2-yn-1-ol obtenu à l'exemple 13(d) avec 340 µl (5,5 mmoles) d'iodure de méthyle, on obtient 600 mg (41%) du composé attendu, sous la forme d'un solide jaune de point de fusion 68-70°C.
1H NMR (CDCl3) d 1,32 (s, 9H), 1,39 (s, 9H), 2,58 (d, 1H, J = 2,2 Hz), 3,47 (s, 3H), 3,82 (s, 3H), 5,34 (d, 1H, J = 2,2 Hz), 7,34 (d, 1H, J = 2,5 Hz), 7,54 (d, 1H, J = 2,5 Hz).

### (b) 4- [3-(3,5-di-tert-butyl-2-méthoxy-phényl)-3-méthoxy-prop-1-ynyl]-benzoate d'éthyle.

De manière analogue à l'exemple 3(c), par réaction de 220 mg (0,8 mmole) du composé obtenu à l'exemple 24(a) avec 220 mg (0,8 mmole) de 4-iodobenzoate d'éthyle, on obtient 260 mg (74%) de 4-[3-(3,5-di-tert-butyl-2-méthoxy-phényl)-3-méthoxy-prop-1-ynyl]-benzoate d'éthyle, sous la forme d'une huile orange.
1H NMR (CDCl3) d 1,33 (s, 9H), 1,38 (t, 3H, J = 7,1 Hz), 1,41 (s, 9H), 3,53 (s, 3H), 3,88 (s, 3H), 4,37 (q, 2H, J = 7,1 Hz), 5,57 (s, 1H), 7,36 (d, 1H, J = 2,5 Hz), 7,51 (d, 1H, J = 8,4 Hz), 7,62 (d, 1H, J = 2,5 Hz), 7,99 (d, 1H, J = 8,4 Hz).

### EXEMPLE 25

### Acide 4-[3-(3,5-di-tert-butyl-2-méthoxy-phényl)-3-méthoxy-prop-1-ynyl]-benzoïque.

De manière analogue à l'exemple 2, à partir de 260 mg (0,6 mmole) du composé obtenu à l'exemple 24(b), on obtient 180 mg (73%) d'acide 4-[3-(3,5-di-tert-butyl-2-méthoxy-phényl)-3-méthoxy-prop-1-ynyl]-benzoique, sous la forme d'une poudre beige de point de fusion 162-164°C.
1H NMR (CDCl3) d 1,33 (s, 9H), 1,41 {s, 9H), 3,54 (s, 3H), 3,88 (s, 3H), 5,58 (s, 1H), 7,36 (d, 1H, J = 2,5 Hz), 7,55 (d, 1H, J = 8,3 Hz), 7,62 (d, 1H, J = 2,5 Hz), 8,06 (d, 1H, J = 8,4 Hz).

### EXEMPLE 26

### 4-[3-(4,4-diméthyl-thiochroman-8-yl)-prop-1-ynyl]-benzoate de méthyle.

De manière analogue à l'exemple 3(c), par réaction de 4,00 g (18,5 mmoles) du composé obtenu à l'exemple 5(c) avec 3,88 g (14,8 mmoles) de 4-iodobenzoate de méthyle, on obtient 1,66 g (25%) de 4-[3-(4,4-diméthyl-thiochroman-8-yl)-prop-1-ynyl]-benzoate de méthyle, sous la forme d'une poudre jaune de point de fusion 92°C.
1H NMR (CDCl3) d 1,35 (s, 6H), 1,96 (t, 2H, J = 6,0 Hz), 3,05 (t, 2H, J = 6,2 Hz), 3,77 (s, 2H), 3,91 (s, 3H), 7,07 (t, 1H, J = 7,7 Hz), 7,33 (d, 1H, J = 7,0 Hz), 7,42 (d, 1H, J = 7,3 Hz), 7,51 (d, 2H, J = 8,4 Hz), 7,97 (d, 2H, J = 8,4 Hz).

### EXEMPLE 27

### 6-[3-(4,4-diméthyl-thiochroman-8-yl)-prop-1-ynyl]-nicotinate d'éthyle.

De manière analogue à l'exemple 3(c), par réaction de 1,00 g (4,6 mmoles) du composé obtenu à l'exemple 5(c) avec 1,41 g (5,1 mmoles) de 6-iodo-pyridine-3-carboxylate d' éthyle, on obtient 50 mg (3%) de 6-[3-(4,4-diméthyl-thiochroman-8-yl)-prop-1-ynyl]-nicotinate d'éthyle, sous la forme d'une huile jaune.
1H NMR (CDCl3) d 1,35 (s, 6H), 1,41 (t, 3H, J = 7,2 Hz), 1,97 (t, 2H, J = 6,0 Hz), 3,06 (t, 2H, J = 6,1 Hz), 3,82 (s, 2H), 4,41 (q, 2H, J = 7,1 Hz), 7,06 (t, 1H, J = 7,7 Hz), 7,34 (d, 1H, J = 7,9 Hz), 7,42 (d, 1H, J = 7,4 Hz), 7,51 (d, 1H, J = 8,2 Hz), 8,23 (dd, 1H, J = 8,1 / 2,1 Hz), 9,16 (d, 1H, J = 1,8 Hz).

### EXEMPLE 28

### 4-[3-(4,4-diméthyl-thiochroman-8-yl)-prop-1-ynyl]-benzaldéhyde.

De manière analogue à l'exemple 3(c), par réaction de 2,00 g (9,3 mmoles) du composé obtenu à l'exemple 5(c) avec 1,88 g (10,2 mmoles) de 4-bromobenzaldéhyde, on obtient 90 mg (5%) de 4-[3-(4,4-diméthyl-thiochroman-8-yl)-prop-1-ynyl]-benzaldéhyde, sous la forme d'une poudre jaune de point de fusion 55-63°C.
1H NMR (CDCl3) d 1,34 (s, 6H), 1,95 (t, 2H, J = 6,1 Hz), 3,05 (t, 2H, J = 6,2 Hz), 7,00 (t, 1H, J = 8,6 Hz), 7,17 (dd, 1H, J = 7,5 / 1,2 Hz), 7,29 (dd, 1H, J = 7,1/1,3 Hz), 7,58 (d, 2H, J = 8,2 Hz), 7,80 (d, 2H, J = 8,2 Hz), 9,94 (s, 1H).

### EXEMPLE 29

### 4-[3-(4,4-diméthyl-thiochroman-8-yl)-prop-1-ynyl]-phénol.

De manière analogue à l'exemple 3(c), par réaction de 1,00 g (4,6 mmoles) du composé obtenu à l'exemple 5(c) avec 880 mg (5,1 mmoles) de 4-bromophénol, on obtient 286 mg (20%) de 4-[3-(4,4-diméthyl-thiochroman-8-yl)-prop-1-ynyl]-phénol, sous la forme d'une poudre jaune de point de fusion 95°C.
1H NMR (CDCl3) d 1,33 (s, 6H), 1,94 (t, 2H, J = 6,0 Hz), 3,03 (t, 2H, J = 6,1 Hz), 3,72 (s, 2H), 6,75 (d, 2H, J = 8,7 Hz), 7,07 (t, 1H, J = 7,7 Hz), 7,27 à 7,34 (m, 3H), 7,46 (d, 1H, J = 7,4 Hz).

### EXEMPLE 30

### 4-[3-(5-tert-butyl-4-hydroxy-biphényl-3-yl)-3-hydroxy-prop-1-ynyl]-benzoate d'éthyle.

Dans un tricol de 100 ml, et sous courant d'azote, on introduit 1,44 g (3,0 mmoles) du composé obtenu à l'exemple 20(h), et 15 ml d'éthanol. On ajoute goutte à goutte 830 µl (15,0 mmoles) d'acide sulfurique concentré. Le milieu réactionnel est agité pendant trois heures à la température ambiante, puis on ajoute de l'eau, extrait par de l'éther éthylique, lave la phase organique à l'eau jusqu'à pH neutre, sèche sur sulfate de magnésium, filtre et évapore les solvants. On recueille 1,25 g (100%) de 4-[3-(5-tert-butyl-4-hydroxy-biphényl-3-yl)-3-hydroxy-prop-1-ynyl]-benzoate d'éthyle, sous la forme d'une huile rougeâtre.
1H NMR (CDCl3) d 1,40 (t, 3H, J = 7,2 Hz), 1,48 (s, 9H), 4,38 (q, 2H, J = 7,2 Hz), 5,69 (s, 1H), 7,31 (d, 1H, J = 7,1 Hz), 7,38 à 7,44 (m, 3H), 7,52 à 7,56 (m, 5H), 7,97 (s, 1H), 8,00 (d, 2H, J = 8,4 Hz).

### EXEMPLE 31

### Acide 4-[3-(5-tert-butyl-4-méthoxy-biphényl-3-yl)-prop-1-ynyl]-benzoïque.

De manière analogue à l'exemple 9, à partir de 700 mg (1,7 mmole) du composé obtenu à l'exemple 23, on obtient 508 mg (75%) d'acide 4-[3-(5-tert-butyl-4-méthoxy-biphényl-3-yl) -prop-1-ynyl] -benzoique, sous la forme d'une poudre blanche de point de fusion 229-231°C.
1H NMR (CDCl3) d 1,45 (s, 9H), 3,89 (s, 3H), 3,94 (s, 2H), 7,34 (d, 1H, J = 7,1 Hz), 7,40 à 7,49 (m, 5H), 7,56 à 7,60 (m, 2H), 7,66 (d, 1H, J = 2,2 Hz), 7,98 (d, 2H, J = 8,3 Hz).

### Exemples de compositions pharmaceutiques et cosmétiques.

Dans les exemples suivants, on a illustré diverses formulations pharmaceutiques et cosmétiques à base des composés actifs selon l'invention.

### A - VOIE ORALE

(a) Comprimé de 0,2 g
   - Composé préparé à l'exemple 2 0,001 g
   - Amidon 0,114 g
   - Phosphate bicalcique 0,020 g
   - Silice 0,020 g
   - Lactose 0,030 g
   - Talc 0,010 g
   - Stéarate de magnésium 0,005 g

   Le composé selon l'exemple 2 peut être avantageusement remplacé par la même quantité d'un des composés selon les exemples 4, 6, 11, 12, 21, 25 ou 31.
(b) Suspension buvable en ampoules de 5 ml
   - Composé préparé à l'exemple 4 0,001 g
   - Glycérine 0,500 g
   - Sorbitol à 70% 0,500 g
   - Saccharinate de sodium 0,010 g
   - p-hydroxybenzoate de méthyle 0,040 g
   - Arôme qs
   - Eau purifiée q.s.p 5 ml

   Le composé selon l'exemple 4 peut être avantageusement remplacé par la même quantité d'un des composés selon les exemples 8, 12, 18 et 19.
(c) Comprimé de 0,8 g
   - Composé de l'exemple 6 0,500 g
   - Amidon prégélatinisé 0,100 g
   - Cellulose microcristalline 0,115 g
   - Lactose 0,075 g
   - Stéarate de magnésium 0,010 g
(d) Suspension buvable en ampoules de 10 ml
   - Composé de l'exemple 6 0,200 g
   - Glycérine 1,000 g
   - Sorbitol à 70% 1,000 g
   - Saccharinate de sodium 0,010 g
   - p-hydroxybenzoate de méthyle 0,080 g
   - Arôme qs
   - Eau purifiée q.s.p 10 ml

   Le composé selon l'exemple 6 peut être avantageusement remplacé par la même quantité d'un des composés selon les exemples 11, 12, 14, 23 ou 25.

### B - VOIE TOPIQUE

(a) Onguent
   - Composé de l'exemple 4 0,020 g
   - Myristate d'isopropyle 81,700 g
   - Huile de vaseline fluide 9,100 g
   - Silice ("Aérosil 200" vendue par DEGUSSA) 9,180 g
(b) Onguent
   - Composé de l'exemple 1 0,300 g
   - Vaseline blanche codex 100 g

   Dans cet exemple, le composé de l'exemple 1 peut être avantageusement remplacé par la même quantité d'un composé selon les exemples 28 et 29.
(c) Crème eau-dans-l'huile non ionique
   - Composé de l'exemple 2 0,100 g
   - Mélange d'alcools de lanoline émulsifs, de cires et d'huiles ("Eucérine anhydre" vendu par BDF) 39,900 g
   - p-hydroxybenzoate de méthyle 0,075 g
   - p-hydroxybenzoate de propyle 0,075 g
   - Eau déminéralisée stérile q.s.p 100 g
(d) Lotion
   - Composé de l'exemple 4 0,100 g
   - Polyéthylène glycol (PEG 400) 69,900 g
   - Ethanol à 95% 30,000 g

   Dans les exemples (c) et (d) ci-dessus, le composé selon l'exemple 4 peut être avantageusement remplacé par la même quantité d'un des composés selon les exemples 6, 9, 11, 14, 21, 23 et 31.
(e) Onguent hydrophobe
   - Composé de l'exemple 2 0,300 g
   - Myristate d'isopropyle 36,400 g
   - Huile de silicone ("Rhodorsil 47V300" vendu par RHONE-POULENC) 36,400 g
   - Cire d'abeille 13,600 g
   - Huile de silicone ("Abil 300.000 cst" vendu par GOLDSCHMIDT) 100 g
(f) Crème huile-dans-l'eau non ionique
   - Composé de l'exemple 5 1,000 g
   - Alcool cétylique 4,000 g
   - Monostéarate de glycérol 2,500 g
   - Stéarate de PEG 50 2,500 g
   - Beurre de karité 9,200 g
   - Propylène glycol 2,000 g
   - p-hydroxybenzoate de méthyle 0,075 g
   - p-hydroxybenzoate de propyle 0,075 g
   - Eau déminéralisée stérile 100 g

   Dans cet exemple, le composé selon l'exemple 5 peut être avantageusement remplacé par la même quantité d'un des composés selon les exemples 7, 10, 13, 15, 17, 20 ou 22.

## Revendications

1. Composés bi-aromatiques reliés par une liaison propynylène ou allénylène, **caractérisés par le fait qu'**ils répondent à la formule générale (I) suivante : 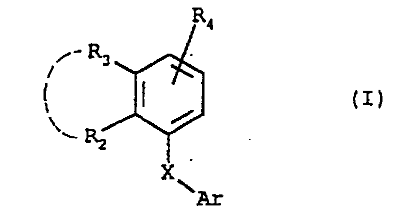 dans laquelle :
Ar représente un radical choisi parmi les formules (a) à (c) suivantes : 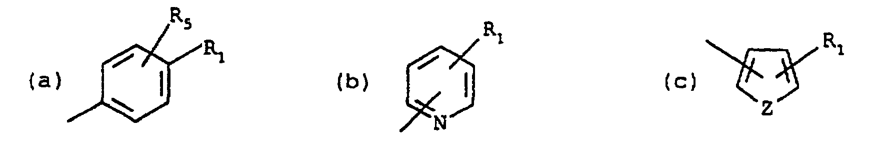 Z étant un atome d'oxygène ou de soufre,
R₁ représente -CH₃, -CH₂-O-R₆, -OR₆ ou -COR₇,
R₂ représente -OR₈, -SR₈ ou un radical ayant de 1 à 6 atomes de carbone et de 1 à 3 atomes d'oxygène ou de soufre si dans ce dernier cas R₄ représente alkyle, linéaire ou ramifié, en C₁-C₂₀ et est en position ortho ou méta par rapport à la liaison X-Ar,
R3 représente alkyle en C₁-C₆ ou
R₂ ou R₃ pris ensemble forment un cycle à 5 ou 6 chaînons éventuellement substitué par au moins un méthyle et/ou éventuellement interrompu par un atome d'oxygène ou de soufre,
R₄ représente H, un halogène, alkyle, linéaire ou ramifié, en C₁-C₂₀, -OR₈, un radical ayant de 1 à 6 atomes de carbone et de 1 à 3 atomes d'oxygène ou de soufre ou un radical aryle,
R₅ représente H, un halogène, alkyle, linéaire ou ramifié, en C₁-C₂₀ ou un radical -OR₈,
R₆ représente H, alkyle en C₁-C₆ ou un radical -COR₉,
R₇ représente H, alkyle en C₁-C₆, 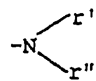 ou -OR₁₀,
R₈ représente H, alkyle en C₁-C₆ ou -COR₉,
R₉ représente alkyle en C₁-C₆,
R₁₀ représente H, alkyle, linéaire ou ramifié, en C₁-C₂₀, alkényle, mono- ou polyhydroxyalkyle, aryle ou aralkyle éventuellement substitué(s) ou un reste de sucre,
r' et r" représentent H, alkyle en C₁-C₆, mono- ou polyhydroxyalkyle, aryle éventuellement substitué, un reste d'aminoacide ou de sucre ou pris ensemble avec l'atome d'azote forment un hétérocycle,
X représente un radical divalent qui, de droite à gauche ou inversement, a pour formule : 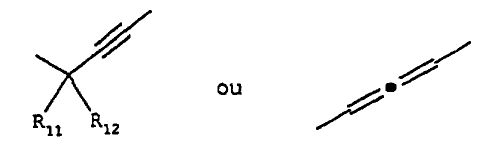
R₁₁ représentant H ou -OR₆, R₆ ayant la même signification que ci-dessus,
R₁₂ représentant H ou alkyle en C₁-C₆, ou
R₁₁ et R₁₂ pris ensemble forment un radical oxo (=O),
et les sels des composés de formule (I) lorsque R₁ représente une fonction acide carboxylique ainsi que les isomères optiques et géométriques des composés de formule (I).

2. Composés selon la revendication 1, **caractérisés en ce qu'**ils se présentent sous forme d'un sel d'un métal alcalin ou alcalino-terreux, ou encore de zinc ou d'une amine organique.

3. Composés selon l'une des revendications 1 ou 2, **caractérisés en ce que** le radical alkyle en C₁-C₆ est choisi dans le groupe constitué par les radicaux méthyle, éthyle, isopropyle, butyle, tertiobutyle, et hexyle.

4. Composés selon l'une quelconque des revendications précédentes, **caractérisés en ce que** le radical alkyle, linéaire ou ramifié, en C₁-C₂₀, est choisi dans le groupe constitué par les radicaux méthyle, éthyle, propyle, isopropyle, hexyle, heptyle, 2-éthyl-hexyle, octyle, nonyle, dodécyle, hexadécyle et octadécyle.

5. Composés selon l'une quelconque des revendications précédentes, **caractérisés en ce que** le radical monohydroxyalkyle est choisi dans le groupe constitué par les radicaux 2-hydroxyéthyle, 2-hydroxypropyle ou 3-hydroxypropyle.

6. Composés selon l'une quelconque des revendications précédentes, **caractérisés en ce que** le radical polyhydroxyalkyle est choisi dans le groupe constitué par les radicaux 2,3-dihydroxypropyle, 2,3,4-trihydroxybutyle, 2,3,4,5-tétrahydroxy-pentyle ou le reste du pentaérythritol.

7. Composés selon l'une quelconque des revendications précédentes **caractérisés par le fait que** le radical contenant de 1 à 6 atomes de carbone et de 1 à 3 atomes d'oxygène ou de soufre est choisi parmi les radicaux méthoxyméthyl éther, méthoxyéthoxyméthyl éther et méthylthiométhyl éther.

8. Composés selon l'une quelconque des revendications précédentes, **caractérisés en ce que** le radical aryle est un radical phényle éventuellement substitué par au moins un atome d'halogène, un hydroxyle ou une fonction nitro, un alkyle inférieur, un radical CF3, un radical amino éventuellement protégé par une fonction acétyle ou éventuellement substitué par un ou deux alkyle(s) inférieur(s), un radical alkoxy ou un radical polyéther.

9. Composés selon l'une quelconque des revendications précédentes, **caractérisés en ce que** le radical aralkyle est choisi dans le groupe constitué par les radicaux benzyle ou phénéthyle éventuellement substitué par au moins un atome d'halogène, un hydroxyle ou une fonction nitro.

10. Composés selon l'une quelconque des revendications précédentes, **caractérisés en ce que** le radical alkényle est choisi dans le groupe constitué par les radicaux contenant de 2 à 5 atomes de carbone et présentant une ou deux insaturation(s) éthylénique(s), en particulier le radical allyle.

11. Composés selon l'une quelconque des revendications précédentes, **caractérisés en ce que** le reste de sucre est choisi dans le groupe constitué par les restes de glucose, de galactose, de mannose ou d'acide glucuronique.

12. Composés selon l'une quelconque des revendications précédentes, **caractérisés en ce que** le reste d'aminoacide est choisi dans le groupe constitué par les restes dérivant de la lysine, de la glycine ou de l'acide aspartique.

13. Composés selon l'une quelconque des revendications précédentes, **caractérisés en ce que** le radical hétérocyclique est choisi dans le groupe constitué par les radicaux pipéridino, morpholino, pyrrolidino ou pipérazino, éventuellement substitué en position 4 par un alkyle en C₁-C₆ ou un mono- ou polyhydroxyalkyle.

14. Composés selon l'une quelconque des revendications précédentes, **caractérisés en ce que** l'atome d'halogène est choisi dans le groupe constitué par le fluor, le chlore et le brome.

15. Composés selon l'une quelconque des revendications précédentes, **caractérisés par le fait qu'**ils répondent à la formule générale suivante : 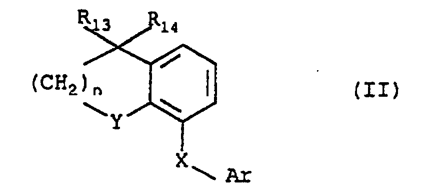 dans laquelle :
Ar représente un radical de formule (a) ou (b) suivante : 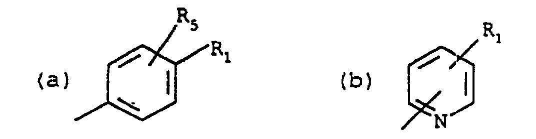
R₁ représente -COR₇,
R₅ et R₇ étant tels que définis à la revendication 1,
X représente un radical divalent qui, de droite à gauche ou inversement, a pour formule : 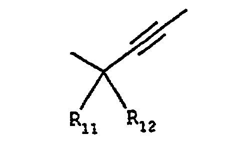
R₁₁ et R₁₂ représentent H,
R₁₃ et R₁₄, identiques ou différents, représentent H ou -CH₃,
Y représente un atome d'oxygène ou de soufre, un radical divalent méthylène, éthylidène ou isopropylidène, et
n est 1 ou 2.

16. Composés selon l'une quelconque des revendications précédentes, **caractérisés par le fait qu'**ils sont pris dans le groupe constitué par :
- 2-Hydroxy-4-[3-(4,4-diméthylchroman-B-yl)-prop-1-ynyl]benzoate de méthyle,
- Acide 2-hydroxy-4-[3-(4,4-diméthylchroman-8-yl)-prop-1-ynyl]benzoïque,
- 2-Hydroxy-4-[3-hydroxy-3-(4,4-diméthylchroman-8-yl)-prop-1-ynyl] benzoate de méthyle,
- Acide 2-hydroxy-4- [3-hydroxy-3-(4,4-diméthylchroman-8-yl)-prop-1-nyl] benzoïque,
- 2-Hydroxy-4-[3-(4,4-diméthylthiochroman-8-yl)-prop-1-nyl] benzoate de méthyle,
- Acide 2-hydroxy-4-[3-(4,4-diméthylthiochroman-8-yl)-prop-1-nyl]benzïque,
- 4-[3-Hydroxy-3-(5,5,8,8-tétraméthyl-3-phényl-5,6,7,8-tétrahydro-naphthalèn-1-yl)-prop-1-ynyl]-benzoate d'éthyle,
- Acide 4-[3-hydroxy-3-(5,5,8,8-tétraméthyl-3-phényl-5,6,7,8-tétrahydro-naphthalèn-1-yl)-prop-1-ynyl]-benzoïque,
- Acide 4-[3-(5,5,8,8-tétraméthyl-3-phényl-5,6,7,8-tétrahydro-naphthalèn-1-yl)-prop-1-ynyl]-benzoïque,
- 4-[3-(4,4-diméthyl-thiochroman-5-yl)-3-hydroxy-prop-1-ynyl]-benzoate d'éthyle,
- Acide 4-[3-(4,4-diméthyl-thiochroman-5-yl)-3-hydroxy-prop-1-ynyl]-benzoïque,
- Acide 4-[3-(4,4-diméthyl-thiochroman-5-yl)-prop-1-ynyl]-benzoïque,
- 4-[3-(3,5-di-tert-butyl-2-méthoxyméthoxy-phényl)-3-hydroxy-prop-1-ynyl]-benzoate d'éthyle,
- Acide 4-[3-(3,5-di-tert-butyl-2-méthoxyméthoxy-phényl)-3-hydroxy-prop-1-ynyl]-benzoïque,
- 4-[3-(3,5-di-tert-butyl-2-hydroxy-phényl)-3-hydroxy-prop-1-ynyl]-benzoate d'éthyle,
- 4-[3-(3,5-di-tert-butyl-2-hydroxy-phényl)-prop-1-ynyl]-benzoate d'éthyle,
- 4-[3-(3,5-di-tert-butyl-2-méthoxy-phényl)-3-hydroxy-prop-1-ynyl]-benzoate d'éthyle,
- Acide 4-[3-(3,5-di-tert-butyl-2-méthoxy-phényl)-3-hydroxy-prop-1-ynyl]-benzoïque,
- Acide 4-[3-(3,5-di-tert-butyl-2-méthoxy-phényl)-prop-1-ynyl]-benzoïque,
- 4-[3-(5-tert-butyl-4-méthoxy-biphényl-3-yl)-3-hydroxy-prop-1-ynyl]-benzoate d'éthyle,
- Acide 4-[3-(5-tert-butyl-4-méthoxy-biphényl-3-yl)-3-hydroxy-prop-1-ynyl]-benzoïque,
- 4-[3-(3,5-di-tert-butyl-2-méthoxy-phényl)-3-méthoxy-prop-1-ynyl]-benzoate d'éthyle,
- Acide 4-[3-(3,5-di-tert-butyl-2-méthoxy-phényl)-3-méthoxy-prop-1-ynyl]-benzoïque,
- 4-[3-(4,4-diméthyl-thiochroman-8-yl)-prop-1-ynyl]-benzoate de méthyle,
- 6- [3-(4,4-diméthyl-thiochroman-8-yl)-prop-1-ynyl]-nicotinate d'éthyle,
- 4-[3-(4,4-diméthyl-thiochroman-8-yl)-prop-1-ynyl]-benzaldéhyde,
- 4-[3-(4,4-diméthyl-thiochroman-8-yl)-prop-1-ynyl]-phénol,
- 4- [3-(5-tert-butyl-4-hydroxy-biphényl-3-yl)-3-hydroxy-prop-1-ynyl]-benzoate d'éthyle,
- Acide 4-[3-(5-tert-butyl-4-méthoxy-biphényl-3-yl)-prop-1-ynyl]-benzoïque,
- Acide 4-[3-(4,4-diméthylthiochroman-8-yl)-prop-1-ynyl]benzoïque,
- Acide 4-[3-(5,6,7,8-tétrahydro-5,5,8,8-tétraméthyl-1-naphtyl)-prop-1-ynyl] benzoïque,
- Acide 2-hydroxy-4-[3-(5,6,7,8-tétrahydro-5,5,8,8-tétraméthyl-1-naphtyl)-prop-1-ynyl]benzoïque,
- 2-Hydroxy-4-[3-hydroxy-3-(4,4-diméthylchroman-8-yl)-prop-1-ynyl] benzoate de méthyle,
- Acide 2-hydroxy-4-[3-hydroxy-3-(4,4-diméthylchroman-8-yl)-prop-1-ynyl] benzoïque,
- Acide 2-hydroxy-4-[3-(4,4-diméthylthiochroman-8-yl)-prop-1-ynyl]benzoïque,
- 4-[3-(4,4-diméthyl-thiochroman-8-yl)-prop-1-ynyl]-benzamide,
- N-éthyl-4-[3-(4,4-diméthyl-thiochroman-8-yl)-prop-1-ynyl]-benzamide,
- N-(4-hydroxyphényl)-4-[3-(4,4-diméthyl-thiochroman-8-yl)-prop-1-ynyl]-benzamide,
- Morpholide de l'acide 4-[3-(4,4-diméthyl-thiochroman-8-yl)-prop-1-ynyl]-benzoïque,
- Acide 4-[3-(4,4-diméthyl-thiochroman-8-yl)-prop-2-ynyl]-benzoïque,
- Acide 4-[3-(5,5,8,8-tetraméthyl-5,6,7,8-tetrahydro-naphthalen-1-yl)-prop-2-ynyl]-benzoïque,
- Acide 4-[3-(4,4-diméthyl-6-phényl-thiochroman-8-yl)-prop-1-ynyl]-benzoique,
- Acide 4-[3-(4,4-diméthyl-6-phényl-chroman-8-yl)-prop-1-ynyl]-benzoique,
- Acide 4-[3-(4,4-diméthyl-6-phényl-thiochroman-8-yl)-prop-2-ynyl]-benzoique,
- Acide 4-[3-(4,4-diméthyl-6-p-tolyl-thiochroman-8-yl)-prop-1-ynyl]-benzoique,
- Acide 4-[3-(5,5,8,8-tétraméthyl-5,6,7,8-tetrahydro-naphthalèn-1-yl)-prop-2-ynyl]-benzoique,
- Acide 4-[3-(5,5,8,8-tétraméthyl-3-p-tolyl-5,6,7,8-tétrahydro-naphthalèn-1-yl)-prop-1-ynyl]-benzoique,
- Acide 4-(3-[3-(4-méthoxy-phényl)-5,5,8,8-tétraméthyl-5,6,7,8-tétrahydro-naphthalèn-1-yl]-prop-1-ynyl)-benzoique,
- Acide 2-hydroxy-4-[3-(5,5,8,8-tétraméthyl-3-p-tolyl-5,6,7,8-tétrahydro-naphthalèn-1-yl)-prop-1-ynyl]-benzoique et
- Acide 3-hydroxy-4-[3-(5,5,8,8-tétramèthyl-3-phényl -5,6,7,8-tétrahydro-naphthalèn-1-yl)-prop-1-ynyl]-benzoique.

17. Composés selon l'une quelconque des revendications précédentes pour une utilisation comme médicament.

18. Composés selon la revendication 17 pour une utilisation comme médicament destiné au traitement des affections dermatologiques, rhumatismales, respiratoires, cardiovasculaires et ophtalmologiques.

19. Utilisation de l'un au moins des composés tels que définis selon l'une quelconque des revendications 1 à 16 pour la préparation d'un médicament destiné au traitement des affections dermatologiques, rhumatismales, respiratoires, cardiovasculaires et ophtalmologiques.

20. Composition pharmaceutique, **caractérisée par le fait qu'**elle comprend, dans un support pharmaceutiquement acceptable, au moins un composé tel que défini selon l'une quelconque des revendications 1 à 16.

21. Composition selon la revendication 21, **caracterisée en ce que** la concentration en au moins un composé selon l'une des revendications 1 à 16 est comprise entre 0.001% et 5% en poids par rapport au poids total de la composition.

22. Composition cosmétique, **caractérisée par le fait qu'**elle contient, dans un support cosmétiquement acceptable, au moins un composé tel que défini selon l'une quelconque des revendications 1 à 16.

23. Composition selon la revendication 22, **caractérisée en ce que** la concentration en au moins un composé selon l'une quelconque des revendications 1 à 16 est comprise entre 0,001 et 3% en poids par rapport au poids total de la composition.

24. Utilisation d'une composition cosmétique telle que définie selon l'une quelconque des revendications 22 ou 23 pour l'hygiène corporelle ou capillaire.

## Patentansprüche

1. Biaromatische Verbindungen, die über eine Propynylen- oder Allenylen-Bindung verbunden sind, **dadurch gekennzeichnet, dass** diese der folgenden allgemeinen Formel (I) entsprechen: 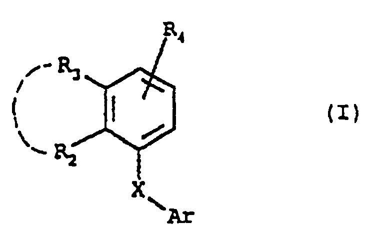 worin:
Ar einen Rest darstellt, ausgewählt unter den folgenden Formeln (a) bis (c): 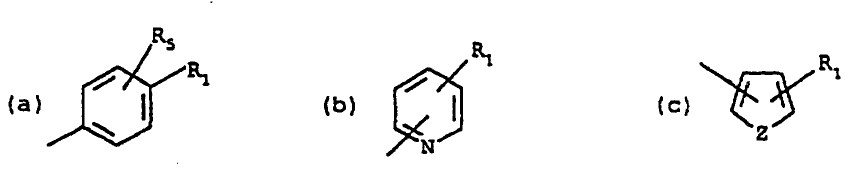
z ein Sauerstoff- oder Schwefelatom ist,
R₁ für -CH₃, -CH₂-O-R₆, -OR₆ oder -COR₇ steht,
R₂ für -OR₈, -SR₈ oder einen Rest mit 1 bis 6 Kohlenstoffatomen und 1 bis 3 Sauerstoff- oder Schwefelatomen steht, wenn in letzterem Fall R₄ für ein lineares oder verzweigtes C₁-C₂₀-Alkyl steht und sich in ortho- oder meta-Position bezogen auf die Bindung X-Ar befindet,
R₃ für ein C₁-C₆-Alkyl steht oder
R₂ oder R₃ zusammengenommen einen Ring mit 5 oder 6 Kettengliedern bilden, gegebenenfalls substituiert durch mindestens ein Methyl und/oder gegebenenfalls unterbrochen durch ein Sauerstoff- oder Schwefelatom,
R₄ für H, ein Halogen, lineares oder verzweigtes C₁-C₂₀-Alkyl, -OR₈, einen Rest mit 1 bis 6 Kohlenstoffatomen und 1 bis 3 Sauerstoff- oder Schwefelatomen oder einen Arylrest steht,
R₅ für H, ein Halogen, lineares oder verzweigtes C₁-C₂₀-Alkyl oder einen Rest -OR₈ steht,
R₆ für H, C₁-C₆-Alkyl oder einen Rest -COR₉ steht,
R₇ für H, C₁-C₆-Alkyl, 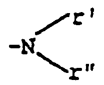 oder -OR₁₀ steht,
R₈ für H, C₁-C₆-Alkyl oder -COR₉ steht,
R₉ für C₁-C₆-Alkyl steht,
R₁₀ für H, lineares oder verzweigtes C₁-C₂₀-Alkyl, Alkenyl, Mono- oder Polyhydroxyalkyl, Aryl oder Aralkyl steht, gegebenenfalls substituiert mit einem Zuckerrest,
r' und r" für H, C₁-C₆-Alkyl, Mono- oder Polyhydroxyalkyl, gegebenenfalls substituiertes Aryl, einen Aminosäurerest oder Zuckerrest stehen oder zusammengenommen mit dem Stickstoffatom einen Heterocyclus bilden,
X für einen divalenten Rest steht, der von rechts nach links oder umgekehrt die Formel aufweist: 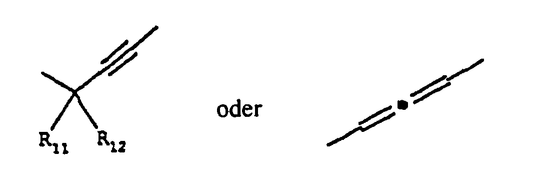
R₁₁ für H oder -OR₆ steht, wobei R₆ dieselbe Bedeutung wie oben hat,
R₁₂ für H oder C₁-C₆-Alkyl steht, oder
worin R₁₁ und R₁₂ zusammengenommen einen Oxorest (=O) bilden,
und die Salze der Verbindungen der Formel (I), wenn R₁ für eine Carbonsäurefunktion steht, wie auch die optischen und geometrischen Isomeren der Verbindungen der Formel (I).

2. Verbindungen gemäß Anspruch 1, **dadurch gekennzeichnet, dass** sie in Form eines Salzes eines Alkalimetalls oder Erdalkalimetalls oder auch von Zink oder einem organischen Amin vorliegen.

3. Verbindungen gemäß einem der Ansprüche 1 oder 2, **dadurch gekennzeichnet, dass** der C₁-C₆-Alkylrest ausgewählt ist aus der Gruppe, bestehend aus den Resten Methyl, Ethyl, Isopropyl, Butyl, tert-Butyl und Hexyl.

4. Verbindungen gemäß einem der vorstehenden Ansprüche, **dadurch gekennzeichnet, dass** der lineare oder verzweigte C₁-C₂₀-Alkylrest ausgewählt ist aus der Gruppe, bestehend aus den Resten Methyl, Ethyl, Propyl, Isopropyl, Hexyl, Heptyl, 2-Ethylhexyl, Octyl, Nonyl, Dodecyl, Hexadecyl und Octadecyl.

5. Verbindungen gemäß einem der vorstehenden Ansprüche, **dadurch gekennzeichnet, dass** der Monohydroxyalkylrest ausgewählt ist aus der Gruppe, bestehend aus den Resten 2-Hydroxyethyl, 2-Hydroxypropyl oder 3-Hydroxypropyl.

6. Verbindungen gemäß einem der vorstehenden Ansprüche, **dadurch gekennzeichnet, dass** der Polyhydroxyalkylrest ausgewählt ist aus der Gruppe, bestehend aus den Resten 2,3-Dihydroxypropyl, 2,3,4-Trihydroxybutyl, 2,3,4,5-Tetrahydroxypentyl oder dem Pentaerythritrest.

7. Verbindungen gemäß einem der vorstehenden Ansprüche, **dadurch gekennzeichnet, dass** der 1 bis 6 Kohlenstoffatome und 1 bis 3 Sauerstoff- oder Schwefelatome enthaltende Rest ausgewählt ist aus den Resten Methoxymethylether, Methoxyethoxymethylether und Methylthiomethylether.

8. Verbindungen gemäß einem der vorstehenden Ansprüche, **dadurch gekennzeichnet, dass** der Arylrest ein Phenylrest ist, gegebenenfalls substituiert durch mindestens ein Halogenatom, ein Hydroxyl- oder eine Nitrofunktion, ein niederes Alkyl, einen CF₃-Rest, einen Aminorest, gegebenenfalls geschützt durch eine Acetylfunktion oder gegebenenfalls substituiert mit einem oder zwei niederen Alkylen, einen Alkoxyrest oder einen Polyetherrest.

9. Verbindungen gemäß einem der vorstehenden Ansprüche, **dadurch gekennzeichnet, dass** der Aralkylrest ausgewählt ist aus der Gruppe, bestehend aus den Resten Benzyl oder Phenethyl, gegebenenfalls substituiert durch mindestens ein Halogenatom, eine Hydroxyl- oder eine Nitrofunktion.

10. Verbindungen gemäß einem der vorstehenden Ansprüche, **dadurch gekennzeichnet, dass** der Alkenylrest ausgewählt ist aus der Gruppe, bestehend aus den Resten, die 2 bis 5 Kohlenstoffatome enthalten und 1 oder 2 ethylenische Unsättigungen aufweisen, insbesondere dem Allylrest.

11. Verbindungen gemäß einem der vorstehenden Ansprüche, **dadurch gekennzeichnet, dass** der Zuckerrest ausgewählt ist aus der Gruppe, bestehend aus den Resten von Glukose, Galactose, Mannose oder Glucuronsäure.

12. Verbindungen gemäß einem der vorstehenden Ansprüche, **dadurch gekennzeichnet, dass** der Aminosäurerest ausgewählt ist aus der Gruppe, bestehend aus den Resten, die sich von Lysin, Glycin oder Asparaginsäure ableiten.

13. Verbindungen gemäß einem der vorstehenden Ansprüche, **dadurch gekennzeichnet, dass** der heterocyclische Rest ausgewählt ist aus der Gruppe, bestehend aus den Resten Piperidino, Morpholino, Pyrrolidino oder Piperazino, gegebenenfalls substituiert in Position 4 durch ein C₁-C₆-Alkyl oder ein Mono- oder Polyhydroxyalkyl.

14. Verbindungen gemäß einem der vorstehenden Ansprüche, **dadurch gekennzeichnet, dass** das Halogenatom ausgewählt ist aus der Gruppe bestehend aus Fluor, Chlor und Brom.

15. Verbindungen gemäß einem der vorstehenden Ansprüche, **dadurch gekennzeichnet, dass** sie der folgenden allgemeinen Formel entsprechen: 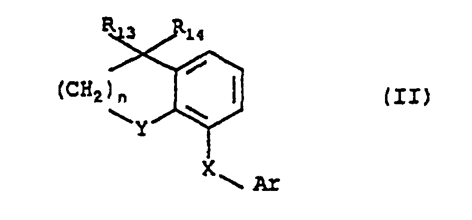 worin:
Ar für einen Rest der folgenden Formel (a) oder (b) steht: 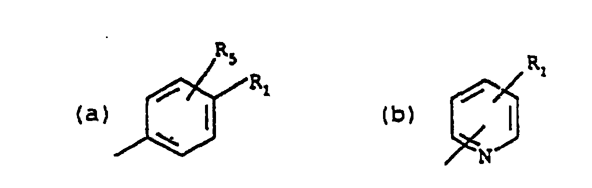
R₁ für -COR₇ steht,
R₅ und R₇ wie in Anspruch 1 definiert sind,
X für einen divalenten Rest steht, der von rechts nach links oder umgekehrt die Formel aufweist: 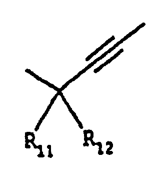
R₁₁ und R₁₂ für H stehen,
R₁₃ und R₁₄, die gleich oder verschieden sind, für H oder -CH₃ stehen,
Y für ein Sauerstoff- oder Schwefelatom, einen zweiwertigen Methylen-, Ethyliden- oder Isopropylidenrest steht und
n 1 oder 2 ist.

16. Verbindungen gemäß einem der vorstehenden Ansprüche, **dadurch gekennzeichnet, dass** sie aus der Gruppe sind, bestehend aus:
- 2-Hydroxy-4-[3-(4,4-dimethylchroman-8-yl)-prop-1-ynyl]-benzoesäuremethylester,
- 2-Hydroxy-4-[3-(4,4-dimethylchroman-8-yl)-prop-1-ynyl]-benzoesäure,
- 2-Hydroxy-4-[3-hydroxy-3-(4,4-dimethylchroman-8-yl)-prop-1-ynyl]-benzoesäuremethylester,
- 2-Hydroxy-4-[3-hydroxy-3-(4,4-dimethylchroman-8-yl)-prop-1-nyl]-benzoesäure,
- 2-Hydroxy-4-[3-(4,4-dimethylthiochroman-8-yl)-prop-1-nyl]-benzoesäuremethylester,
- 2-Hydroxy-4-[3-(4,4-dimethylthiochroman-8-yl)-prop-1-nyl]-benzoesäure,
- 4-[3-Hydroxy-3-(5,5,8,8-tetramethyl-3-phenyl-5,6,7,8-tetrahydronaphthalin-1-yl)-prop-1-ynyl]-benzoesäureethylester,
- 4-[3-Hydroxy-3-(5,5,8,8-tetramethyl-3-phenyl-5,6,7,8-tetrahydronaphthalin-1-yl)-prop-1-ynyl]-benzoesäure,
- 4-[3-(5,5,8,8-Tetramethyl-3-phenyl-5,6,7,8-tetrahydronaphthalin-1-yl)-prop-1-ynyl]-benzoesäure,
- 4-[3-(4,4-Dimethylthiochroman-5-yl)-3-hydroxyprop-1-ynyl]-benzoesäureethylester,
- 4-[3-(4,4-Dimethylthiochroman-5-yl)-3-hydroxyprop-1-ynyl]-benzoesäure,
- 4-[3-(4,4-Dimethylthiochroman-5-yl)-prop-1-ynyl]-benzoesäure,
- 4-[3-(3,5-Di-tert-butyl-2-methoxymethoxyphenyl)-3-hydroxyprop-1-ynyl]-benzoesäureethylester,
- 4-[3-(3,5-Di-tert-butyl-2-methoxymethoxyphenyl)-3-hydroxyprop-1-ynyl]-benzoesäure,
- 4-[3-(3,5-Di-tert-butyl-2-hydroxyphenyl)-3-hydroxyprop-1-ynyl]-benzoesäureethylester,
- 4-[3-(3,5-Di-tert-butyl-2-hydroxyphenyl)-prop-1-ynyl]-benzoesäureethylester,
- 4-[3-(3,5-Di-tert-butyl-2-methoxyphenyl)-3-hydroxyprop-1-ynyl]-benzoesäureethylester,
- 4-[3-(3,5-Di-tert-butyl-2-methoxyphenyl)-3-hydroxyprop-1-ynyl]-benzoesäure,
- 4-[3-(3,5-Di-tert-butyl-2-methoxyphenyl)-prop-1-ynyl]-benzoesäure,
- 4-[3-(5-tert-Butyl-4-methoxybiphenyl-3-yl)-3-hydroxyprop-1-ynyl]-benzoesäureethylester,
- 4-[3-(5-tert-Butyl-4-methoxybiphenyl-3-yl)-3-hydroxyprop-1-ynyl]-benzoesäure,
- 4-[3-(3,5-Di-tert-butyl-2-methoxyphenyl)-3-methoxyprop-1-ynyl]-benzoesäureethylester,
- 4-[3-(3,5-Di-tert-butyl-2-methoxyphenyl)-3-methoxyprop-1-ynyl]-benzoesäure,
- 4-[3-(4,4-Dimethylthiochroman-8-yl)-prop-1-ynyl]-benzoesäuremethylester,
- 6-[3-(4,4-Dimethylthiochroman-8-yl)-prop-1-ynyl]-nicotinsäureethylester,
- 4-[3-(4,4-Dimethylthiochroman-8-yl)-prop-1-ynyl]-benzaldehyd,
- 4-[3-(4,4-Dimethylthiochroman-8-yl)-prop-1-ynyl]-phenol,
- 4-[3-(5-tert-Butyl-4-hydroxybiphenyl-3-yl)-3-hydroxyprop-1-ynyl]-benzoesäureethylester,
- 4-[3-(5-tert-Butyl-4-methoxybiphenyl-3-yl)-prop-1-ynyl]-benzoesäure,
- 4-[3-(4,4-Dimethylthiochroman-8-yl)-prop-1-ynyl]-benzoesäure,
- 4-[3-(5,6,7,8-Tetrahydro-5,5,8,8-tetramethyl-1-naphthyl)-prop-1-ynyl]-benzoesäure,
- 2-Hydroxy-4-[3-(5,6,7,8-tetrahydro-5,5,8,8-tetramethyl-1-naphthyl)-prop-1-ynyl]-benzoesäure,
- 2-Hydroxy-4-[3-hydroxy-3-(4,4-dimethylchroman-8-yl)-prop-1-ynyl]-benzoesäuremethylester,
- 2-Hydroxy-4-[3-hydroxy-3-(4,4-dimethylchroman-8-yl)-prop-1-ynyl]-benzoesäure,
- 2-Hydroxy-4-[3-(4,4-dimethylthiochroman-8-yl)-prop-1-ynyl]-benzoesäure,
- 4-[3-(4,4-dimethylthiochroman-8-yl)-prop-1-ynyl]-benzamid,
- N-Ethyl-4-[3-(4,4-dimethlythiochroman-8-yl)-prop-1-ynyl]-benzamid,
- N-(4-Hydroxyphenyl)-4-[3-(4,4-dimethylthiochroman-8-yl)-prop-1-ynyl]-benzamid,
- 4-[3-(4,4-Dimethylthiochroman-8-yl)-prop-1-ynyl]-benzoesäure-Morpholid,
- 4-[3-(4,4-Dimethylthiochroman-8-yl)-prop-1-ynyl]-benzoesäure,
- 4-[3-(5,5,8,8-Tetramethyl-5,6,7,8-tretahydronaphthalin-1-yl)-prop-1-ynyl]-benzoesäure,
- 4-[3-(4,4-Dimethyl-6-phenylthiochroman-8-yl)-prop-1-ynyl]-benzoesäure,
- 4-[3-(4,4-Dimethyl-6-phenylchroman-8-yl)-prop-1-ynyl]-benzoesäure,
- 4-[3-(4,4-Dimethyl-6-phenylthiochroman-8-yl)-prop-2-ynyl]-benzoesäure,
- 4-[3-(4,4-Dimethyl-6-p-tolythiochroman-8-yl)-prop-1-ynyl]-benzoesäure,
- 4-[3-(5,5,8,8-Tetramethyl-5,6,7,8-tetrahydronaphthalin-1-yl)-prop-2-ynyl]-benzoesäure,
- 4-[3-(5,5,8,8-Tetramethyl-3-p-tolyl-5,6,7,8-tetrahydronaphthalin-1-yl)-prop-1-ynyl]-benzoesäure,
- 4-(3-[3-(4-Methoxyphenyl)-5,5,8,8-tetramethyl-5,6,7,8-tetrahydronaphthalin-1-yl)-prop-1-ynyl]-benzoesäure,
- 2-Hydroxy-4-[3-(5,5,8,8-tetramethyl-3-p-tolyl-5,6,7,8-tetrahydronaphthalin-1-yl)-prop-1-ynyl]-benzoesäure und
- 3-Hydroxy-4-[3-(5,5,8,8-tetramethyl-3-phenyl-5,6,7,8-tetrahydronaphthalin-1-yl)-prop-1-ynyl]-benzoesäure.

17. Verbindungen gemäß einem der vorstehenden Ansprüche für die Verwendung als Medikament.

18. Verbindungen gemäß Anspruch 17 für eine Verwendung als Medikament, bestimmt zur Behandlung von dermatologischen, rheumatischen, respiratorischen, kardiovaskulären und ophtalmologischen Beschwerden.

19. Verwendung mindestens einer der Verbindungen, wie sie gemäß einem der Ansprüche 1 bis 16 definiert sind, für die Herstellung eines Medikaments bestimmt zur Behandlung von dermatologischen, rheumatischen, respiratorischen, kardiovaskulären und ophtalmologischen Beschwerden.

20. Pharmazeutische Zubereitung, **dadurch gekennzeichnet, dass** sie in einem pharmazeutisch annehmbaren Träger mindestens eine Verbindung enthält, wie sie in einem der Ansprüche 1 bis 16 definiert ist.

21. Zubereitung gemäß Anspruch 21, **dadurch gekennzeichnet, dass** die Konzentration der mindestens einen Verbindung gemäß einem der Ansprüche 1 bis 16 zwischen 0,001 % und 5 Gew.-% liegt, bezogen auf das Gesamtgewicht der Zubereitung.

22. Kosmetische Zubereitung, **dadurch gekennzeichnet, dass** sie in einem kosmetisch annehmbaren Träger mindestens eine Verbindung enthält, wie sie in einem der Ansprüche 1 bis 16 definiert ist.

23. Zubereitung gemäß Anspruch 22, **dadurch gekennzeichnet, dass** die Konzentration der mindestens einen Verbindung gemäß einem der Ansprüche 1 bis 16 zwischen 0,001 und 3 Gew.-% liegt, bezogen auf das Gesamtgewicht der Zubereitung.

24. Verwendung einer kosmetischen Zubereitung, wie sie in einem der Ansprüche 22 oder 23 definiert ist, für die Körper- oder Haarhygiene.

## Claims

1. Biaromatic compounds connected by a propynylene or allenylene bond, **characterized in that** they correspond to the following general formula (I): 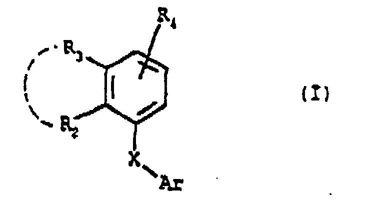 in which:
Ar represents a radical chosen from the following formulae (a) to (c): 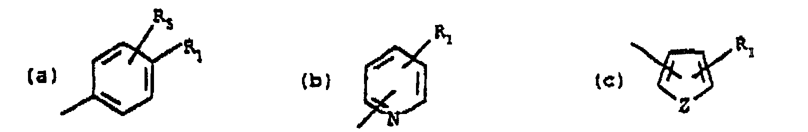 Z being an oxygen or sulphur atom,
R₁ represents -CH₃, -CH₂-O-R₆, -OR₆ or -COR₇,
R₂ represents -OR₈, -SR₈ or a radical having from 1 to 6 carbon atoms and from 1 to 3 oxygen or sulphur atoms, if, in the latter case, R₄ represents linear or branched C₁-C₂₀ alkyl and is at the ortho or meta position with respect to the X-Ar bond,
R₃ represents C₁-C₆ alkyl, or
R₂ and R₃, taken together, form a 5- or 6-membered ring optionally substituted by at least one methyl and/or optionally interrupted by an oxygen or sulphur atom,
R₄ represents H, a halogen, linear or branched C₁-C₂₀ alkyl, -OR₈, a radical having from 1 to 6 carbon atoms and from 1 to 3 oxygen or sulphur atoms, or an aryl radical,
R₅ represents H, a halogen, linear or branched C₁-C₂₀ alkyl or an -OR₈ radical,
R₆ represents H, C₁-C₆ alkyl or a -COR₉ radical,
R₇ represents H, C₁-C₆ alkyl, 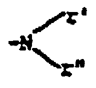 or -OR₁₀,
R₈ represents H, C₁-C₆ alkyl or -COR₉,
R₉ represents C₁-C₆ alkyl,
R₁₀ represents H, C₁-C₂₀ alkyl, which can be linear or branched, alkenyl, mono- or polyhydroxyalkyl, optionally substituted aryl or aralkyl, or a sugar residue,
r' and r" represent H, C₁-C₆ alkyl, mono- or polyhydroxyalkyl, optionally substituted aryl, or an amino acid or sugar residue or, taken together with the nitrogen atom, form a heterocycle,
X represents a divalent radical which, from right to left or vice versa, has the formula: 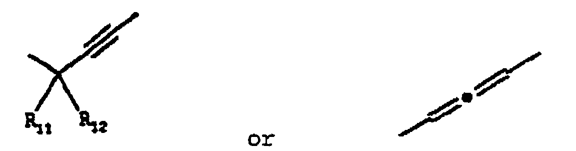
R₁₁ representing H or -OR₆, R₆ having the same meaning as above,
R₁₂ representing H or C₁-C₆ alkyl, or
R₁₁ and R₁₂, taken together, form an oxo (=O) radical,
and the salts of the compounds of formula (I), when R₁ represents a carboxylic acid functional group, and the optical and geometrical isomers of the compounds of formula (I).

2. Compounds according to Claim 1, **characterized in that** they are provided in the form of a salt of an alkali metal or alkaline earth metal or alternatively of zinc or of an organic amine,

3. Compounds according to either of Claims 1 and 2, **characterized in that** the C₁-C₆ alkyl radical is chosen from the group consisting of the methyl, ethyl, isopropyl, butyl, tert-butyl and hexyl radicals.

4. Compounds according to any one of the preceding claims, **characterized in that** the C₁-C₂₀ alkyl radical, which can be linear or branched, is chosen from the group consisting of the methyl, ethyl, propyl, isopropyl, hexyl, heptyl, 2-ethylhexyl, octyl, nonyl, dodecyl, hexadecyl and octadecyl radicals.

5. Compounds according to any one of the preceding claims, **characterized in that** the monohydroxyalkyl radical is chosen from the group consisting of the 2-hydroxyethyl, 2-hydroxypropyl or 3-hydroxypropyl radicals.

6. Compounds according to any one of the preceding claims, **characterized in that** the polyhydroxyalkyl radical is chosen from the group consisting of the 2,3-dihydroxypropyl, 2,3,4-trihydroxybutyl or 2,3,4,5-tetrahydroxypentyl radicals or the pentaerythritol residue.

7. Compounds according to any one of the preceding claims, **characterized in that** the radical comprising from 1 to 6 carbon atoms and from 1 to 3 oxygen or sulphur atoms is chosen from the methoxymethyl ether, methoxyethoxymethyl ether and methylthiomethyl ether radicals.

8. Compounds according to any one of the preceding claims, **characterized in that** the aryl radical is a phenyl radical optionally substituted by at least one halogen atom, one hydroxyl, one nitro functional group, one lower alkyl, one CF₃ radical, one amino radical optionally protected by an acetyl functional group or optionally substituted by one or two lower alkyl(s), one alkoxy radical or one polyether radical.

9. Compounds according to any one of the preceding claims, **characterized in that** the aralkyl radical is chosen from the group consisting of the benzyl or phenethyl radical optionally substituted by at least one halogen atom, one hydroxyl or one nitro functional group.

10. Compounds according to any one of the preceding claims, **characterized in that** the alkenyl radical is chosen from the group consisting of the radicals comprising from 2 to 5 carbon atoms and exhibiting one or two ethylenic unsaturation(s), in particular the allyl radical.

11. Compounds according to any one of the preceding claims, **characterized in that** the sugar residue is chosen from the group consisting of the glucose, galactose, mannose or glucuronic acid residues.

12. Compounds according to any one of the preceding claims, **characterized in that** the amino acid residue is chosen from the group consisting of the residues deriving from lysine, glycine or aspartic acid.

13. Compounds according to any one of the preceding claims, **characterized in that** the heterocyclic radical is chosen from the group consisting of the piperidino radical, the morpholino radical, the pyrrolidino radical or the piperazino radical optionally substituted at the 4-position by a C₁-C₆ alkyl or a mono- or polyhydroxyalkyl.

14. Compounds according to any one of the preceding claims, **characterized in that** the halogen atom is chosen from the group consisting of fluorine, chlorine and bromine.

15. Compounds according to any one of the preceding claims, **characterized in that** they correspond to the following general formula: 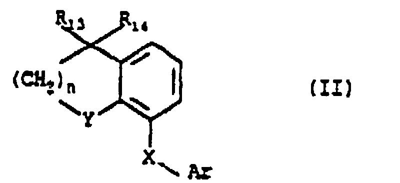 in which:
Ar represents a radical of following formula (a) or (b): 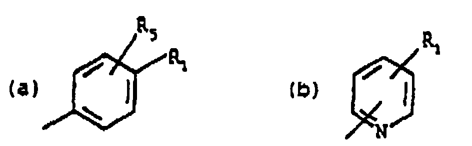
R₁ represents -COR₇,
R₅ and R₇ being as defined in Claim 1,
X represents a divalent radical which, from right to left or vice versa, has the formula: 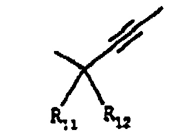
R₁₁ and R₁₂ represent H,
R₁₃ and R₁₄, which are identical or different, represent H or -CH₃,
Y represents an oxygen or sulphur atom or a methylene, ethylidene or isopropylidene divalent radical, and
n is 1 or 2.

16. Compounds according to any one of the preceding claims, **characterized in that** they are taken from the group consisting of:
- Methyl 2-hydroxy-4-[3-(4,4-dimethylchroman-8-yl)prop-1-ynyl]benzoate,
- 2-Hydroxy-4-[3-(4,4-dimethylchroman-8-yl)-prop-1-ynyl]benzoic acid,
- Methyl 2-hydroxy-4-[3-hydroxy-3-(4,4-dimethylchroman-8-yl)prop-1-ynyl]benzoate,
- 2-Hydroxy-4-[3-hydroxy-3-(4,4-dimethylchroman-8-yl)prop-1-ynyl]benzoic acid,
- Methyl 2-hydroxy-4-[3-(4,4-dimethylthiochroman-8-yl)prop-1-ynyl]benzoate,
- 2-Hydroxy-4-[3-(4,4-dimethylthiochroman-8-yl)prop-1-ynyl]benzoic acid,
- Ethyl 4-[3-hydroxy-3-(5,5,8,8-tetramethyl-3-phenyl-5,6,7,8-tetrahydronaphth-1-yl)prop-1-ynyl]-benzoate,
- 4-[3-Hydroxy-3-(5,5,8,8-tetramethyl-3-phenyl-5,6,7,8-tetrahydronaphth-l-yl)prop-l-ynyl]benzoic acid,
- 4-[3-(5,5,8,8-Tetramethyl-3-phenyl-5,6,7,8-tetrahydronaphth-1-yl)prop-1-ynyl]benzoic acid,
- Ethyl 4-[3-(4,4-dimethylthiochroman-5-yl)-3-hydroxyprop-1-ynyl]benzoate,
- 4-[3-(4,4-Dimethylthiochroman-5-yl)-3-hydroxyprop-1-ynyl]benzoic acid,
- 4-[3-(4,4-Dimethylthiochroman-5-yl)prop-1-ynyl]benzoic acid,
- Ethyl 4-[3-(3,5-di-tert-butyl-2-(methoxymethoxy)phenyl)-3-hydroxyprop-1-ynyl]benzoate,
- 4-[3-(3,5-Di-tert-butyl-2-(methoxymethoxy)-phenyl) -3-hydroxyprop-1-ynyl]benzoic acid,
- Ethyl 4-[3-(3,5-di-tert-butyl-2-hydroxyphenyl)-3-hydroxyprop-1-ynyl]benzoate,
- Ethyl 4-[3-(3,5-di-tert-butyl-2-hydroxyphenyl)prop-1-ynyl]benzoate,
- Ethyl 4-[3-(3,5-di-tert-butyl-2-methoxyphenyl)-3-hydroxyprop-1-ynyl]benzoate,
- 4-[3-(3,5-Di-tert-butyl-2-methoxyphenyl)-3-hydroxyprop-1-ynyl]benzoic acid,
- 4-[3-(3,5-Di-tert-butyl-2-methoxyphenyl)prop-1-ynyl]benzoic acid,
- Ethyl 4-[3-(3,5-di-tert-butyl-2-methoxyphenyl)-3-methoxyprop-1-ynyl]benzoate,
- 4-[3-(3,5-Di-tert-butyl-2-methoxyphenyl)-3-methoxyprop-1-ynyl]benzoic acid,
- Methyl 4-[3-(4,4-dimethylthiochroman-8-yl)-prop-1-ynyl]benzoate,
- Ethyl 6-[3-(4,4-dimethylthiochroman-8-yl)-prop-1-ynyl]nicotinate,
- 4-[3-(4,4-Dimethylthiochroman-8-yl)prop-1-ynyl]benzaldehyde,
- 4-[3-(4,4-Dimethylthiochroman-8-yl)prop-1-ynyl]phenol,
- Ethyl 4-[3-(5-tert-butyl-4-hydroxybiphenyl-3-yl)-3-hydroxyprop-1-ynyl]benzoate,
- 4-[3-(5-tert-Butyl-4-methoxybiphenyl-3-yl)prop-1-ynyl]benzoic acid,
- 4-[3-(4,4-Dimethylthiochroman-8-yl)prop-1-ynyl]benzoic acid,
- 4-[3-(5,6,7,8-Tetrahydro-5,5,8,8-tetramethyl-1-naphthyl)prop-1-ynyl]benzoic acid,
- 2-Hydroxy-4-[3-(5,6,7,8-tetrahydro-5,5,8,8-tetramethyl-1-naphthyl)prop-1-ynyl]benzoic acid,
- Methyl 2-hydroxy-4-[3-hydroxy-3-(4,4-dimethylchroman-8-yl)prop-1-ynyl]benzoate,
- 2-Hydroxy-4-[3-hydroxy-3-(4,4-dimethylchroman-8-yl)prop-1-ynyl]benzoic acid,
- 2-Hydroxy-4-[3-(4,4-dimethylthiochroman-8-yl)prop-1-ynyl]benzoic acid,
- 4-[3-(4,4-Dimethylthiochroman-8-yl)prop-1-ynyl]benzamide,
- N-Ethyl-4- [3- (4,4-dimethylthiochroman-8-yl)prop-1-ynyl]benzamide,
- N-(4-Hydroxyphenyl)-4-[3-(4,4-dimethylthiochroman-8-yl)prop-1-ynyl]benzamide,
- 4-[3-(4,4-Dimethylthiochroman-8-yl)prop-1-ynyl]benzoic acid morpholide,
- 4-[3-(4,4-Dimethylthiochroman-8-yl)prop-2-ynyl]benzoic acid,
- 4-[3-(5,5,8,8-Tetramethyl-5,6,7,8-tetrahydronaphth-1-yl)prop-2-ynyl]benzoic acid,
- 4-[3-(4,4-Dimethyl-6-phenylthiochroman-8-yl)prop-1-ynyl]benzoic acid,
- 4-[3-(4,4-Dimethyl-6-phenylchroman-8-yl)prop-1-ynyl]benzoic acid,
- 4-[3-(4,4-Dimethyl-6-phenylthiochroman-8-yl)prop-2-ynyl]benzoic acid,
- 4-[3-(4,4-Dimethyl-6-(p-tolyl)thiochroman-8-yl)prop-1-ynyl]benzoic acid,
- 4-[3-(5,5,8,8-Tetramethyl-5,6,7,8-tetrahydronaphth-1-yl)prop-2-ynyl]benzoic acid,
- 4-[3-(5,5,8,8-Tetramethyl-3-(p-tolyl)-5,6,7,8-tetrahydronaphth-1-yl)prop-1-ynyl]benzoic acid,
- 4-(3-[3-(4-Methoxyphenyl)-5,5,8,8-tetramethyl-5,6,7,8-tetrahydronaphth-1-yl]prop-1-ynyl)-benzoic acid,
- 2-Hydroxy-4-[3-(5,5,8,8-tetramethyl-3-(p-tolyl)-5,6,7,8-tetrahydronaphth-1-yl)prop-1-ynyl]-benzoic acid, and
- 3-Hydroxy-4-[3-(5,5,8,8-tetramethyl-3-phenyl-5,6,7,8-tetrahydronaphth-1-yl)prop-1-ynyl]benzoic acid.

17. Compounds according to any one of the preceding claims, for use as medicament.

18. Compounds according to Claim 17 for use as medicament, which medicament is intended for the treatment of dermatological, rheumatic, respiratory, cardiovascular and ophthalmological conditions.

19. Use of at least one of the compounds as defined according to any one of Claims 1 to 16 for the preparation of a medicament intended for the treatment of dermatological, rheumatic, respiratory, cardiovascular and opthalmological conditions.

20. Pharmaceutical composition, **characterized in that** it comprises, in a pharmaceutically acceptable vehicle, at least one compound as defined according to any one of Claims 1 to 16.

21. Composition according to Claim 20, **characterized in that** the concentration of at least one compound according to one of Claims 1 to 16 is between 0.001% and 5% by weight with respect to the total weight of the composition.

22. Cosmetic composition, **characterized in that** it comprises, in a cosmetically acceptable vehicle, at least one compound as defined according to any one of Claims 1 to 16.

23. Composition according to Claim 22, **characterized in that** the concentration of at least one compound according to any one of Claims 1 to 16 is between 0.001 and 3% by weight with respect to the total weight of the composition.

24. Use of a cosmetic composition as defined according to either one of Claims 22 and 23 for body or hair hygiene.
